(19) Europäisches Patentamt
European Patent Office
Office européen des brevets

(11) EP 2 752 664 A1

(12) **EUROPEAN PATENT APPLICATION**

(43) Date of publication:
**09.07.2014 Bulletin 2014/28**

(51) Int Cl.:
***G01N 33/543*** *(2006.01)*

(21) Application number: **13150432.6**

(22) Date of filing: **07.01.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Max-Planck-Gesellschaft zur Förderung der Wissenschaften e.V.**
**80539 München (DE)**

(72) Inventors:
- **Schmidt, Stephan**
  **14471 Postdam (DE)**
- **Hartmann, Laura**
  **14471 Postdam (DE)**
- **Pussak, Daniel**
  **13629 Berlin (DE)**
- **Pompe, Tilo**
  **01217 Dresden (DE)**

(74) Representative: **Arth, Hans-Lothar**
**ABK Patent Attorneys**
**Jasminweg 9**
**14052 Berlin (DE)**

# (54) Label-free method for the detection of analytes

(57) The present invention relates to method for the detection and/or characterization of an analyte based on determining and preferably optical determining the alteration of mechanical deformation of hydrogel particles and kits suitable for this method.

**Figure 3**

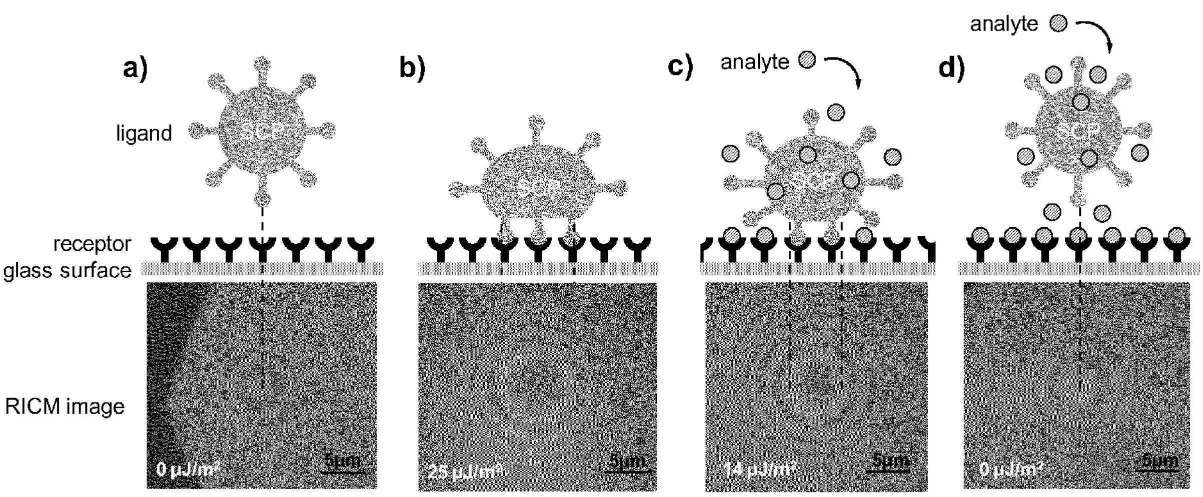


EP 2 752 664 A1

Printed by Jouve, 75001 PARIS (FR)

## Description

The present invention relates to method for the detection and/or characterization of an analyte based on determining and preferably optical determining of the alteration of mechanical deformation of hydrogel particles and kits suitable for this method.

## Background of the invention

The demand for robust and cost-effective analysis in the fields of medical diagnostics and drug screening has risen in recent years. The main reason is for example the increase in allergies, infectious diseases, without the option of preventive vaccination, as well as the growing demand for personalized therapy approaches. In addition to the highest possible accuracy, the economic factor plays a crucial role, as the cost of the analysis should be cut drastically due to rising health care costs. Apart from the human diagnostics also in the fields of veterinary diagnostics, biotechnology, agricultural sciences, and security screening for biological toxins new methods for the detection and characterization of analytes being cost effective and allow rapid analysis are needed.

Biosensors based on surface plasmon resonance (SPR), impedance spectroscopy or quartz crystal microbalance (QCM) have become standard screening methods in the pharmaceutical industry and in research settings. The advantage of these label free techniques lies in their ability to probe biomolecular interactions with high selectivity without changing the binding affinity of the analyte molecules. In general it is the aim to use so called label-free detection methods. Thereby the signal is directly triggered by the binding event, thus avoiding measurement artifacts and possible annoying modifications of the ligand and target molecule. Biosensors detect biochemical interaction, e.g. the interaction between antigen and antibody, or in general, the interaction between the ligand and a target molecule. These specific interactions on a biosensor lead to change of a physical quantity that is converted into a measurable signal.

For example, WO 90/011510 discloses an assay technique involving immobilizing an enzyme on a solid surface and causing the enzyme to catalyze formation of a reaction product. The solid surface is provided by metal film on a glass prism, and deposition of the reaction product is monitored by surface plasmon resonance spectroscopy. The analyte may be a substrate for the immobilized enzyme, or may be part of another enzyme system which generates such a substrate. Besides the plasmon resonance (SPR) also other methods quantifying a physical effect used today like change of impedance or mechanical vibrations. Each of these methods uses a sensitive layer (also called biochip) the active ingredient or the analyte binds to, thereby changing the measured physical variation. The choice of the measurement principle is essential for the sensitivity of the biosensor.

The general developments in this field are directed towards more affordable sensors with increased throughput and enhanced selectivity and sensitivity. Selectivity and sensitivity are still limited in particular for analyte compounds having dielectric constants close to water. Carbohydrates belong to this class of analytes and are exceptionally difficult to analyze because they also show comparatively low affinity and selectivity to their receptors. On the other hand, direct force-based detection via atomic force microscope (AFM), optical traps, or the like offer increased sensitivity and also precise information on the nature of the interaction, e.g. discrimination between specific and unspecific binding. Affinity biosensors and drug screening applications could therefore benefit from force-based detection, however, this approach has not been developed further due to the low throughput of the involved techniques, their expensive instrumentation and the high experimental effort involved with them.

Said label-free methods of the art are currently used primarily in drug development (drug screening) or in basic biochemically research but hardly used in medical diagnostics. This is due to the fact that these methods are very expensive and not miniaturized. Secondly, there are serious technical limitations especially for medical-diagnostic specimens. Complex media such as blood serum cause low selectivity, because of non-specific binding events on the biochip by the multiplicity of interacting sample components. Commonly used in diagnostics are various methods using specific antibodies for the detection of different analytes are known, like enzyme linked immunosorbent assay (ELISA) or assays based on Förster (fluorescence) resonance energy transfer (FRET). These are theoretically very specific for any analyte but practically dye labels used within theses assays as signaling molecules may interact non-specifically, can cause cross reactions and false diagnostic results.

The present invention aims therefore at providing a simple method which can be applied for the detection of any substances, as well as at providing suitable compounds for said method.

It is the objective of the present invention to provide a method for the detection and/or characterization of an analyte based on determining and preferably optical determining the alteration of mechanical deformation of hydrogel particles which also allows detecting specific binding of low molecular weight species as well as analytes with water-like dielectric constant. Thereby the method of the present invention enables surprisingly the detection of binding forces with a cost-effective optical technique.

The objective of the present invention is solved by the teaching of the independent claims. Further advantageous features, aspects and details of the invention are evident from the dependent claims, the description, the figures, and

the examples of the present application.

**Brief description of the invention**

The present invention refers to a label-free assay for the detection and/or characterization of an analyte with target molecule functionalized hydrogel particles (HPs) as sensors. The methods of the invention use a new force-based detection technique that can be easily adapted to construct various biosensors for high throughput applications. As detection principle deformable hydrogel particles and the JKR model are used, which relates adhesion induced mechanical deformation of HPs to their surface energy. The HPs' surface energy and analyte induced variations serves as physical detection signal and is analogous to the resonance frequencies measured with SPR or QCM biosensors. The inventive method is characterized by a direct preferably optical detection without the use of labeling reagents, like chromophores or cantilever. Furthermore, the technology promises improved properties and efficiency compared to affinity-based biosensors, e.g. surface plasmon resonance (SPR, Biacore ™) currently used.

**Description of the invention**

The present invention relates to a method for the detection and/or characterization of an analyte based on determining the alteration of mechanical deformation of hydrogel particles preferably forces based mechanical deformation of hydrogel particles. Determining the alteration is preferably done by optical methods, in particular using an inverted light microscope in reflection interference contrast microscopy (RICM) mode.

Another embodiment of the present invention relates to a method for the detection and/or characterization of an analyte, comprising or consisting of the following steps:

a) providing a deformable, target molecule functionalized hydrogel particle
b) providing a solid carrier with immobilized ligand on its surface
c) providing an analyte to be tested
d) forming a complex between the deformable, target molecule functionalized hydrogel particle and the solid carrier with immobilized ligand by interaction of the receptor and the ligand
e) addition of the analyte and
f) determining the alteration of mechanical deformation of hydrogel particles of step a).

Further embodiment of the present invention relates to a method for the detection and/or characterization of an analyte, comprising or consisting of the following steps:

a) providing a deformable, ligand functionalized hydrogel particle
b) providing a solid carrier with immobilized target molecule on its surface
c) providing an analyte to be tested
d) forming a complex between the deformable, target molecule functionalized hydrogel particle and the solid carrier with immobilized ligand by binding of the receptor and the ligand
e) addition of the analyte and
f) determining the alteration of mechanical deformation of hydrogel particles of step a).

Preferably the present invention relates to a method for the detection and/or characterization of an analyte, comprising further step e)':

e') incubating under conditions sufficient to allow the analyte to bind to the target molecule and replace the ligand in the complex of step d), in case the analyte is able to bind to the target molecule.

The inventive method refers to an assay completely practicable *in vitro.* Within this method the deformable functionalized hydrogel particle represent a colloidal probe having covalently attached a number of a specific target molecule, like a receptor. Alternatively, not the target molecule but the ligand is bound to the hydrogel particle serving as colloidal probe. Therefore the terms "deformable functionalized hydrogel particle", "functionalized hydrogel particle" and "hydrogel particle" used herein refer to deformable, target molecule or ligand functionalized hydrogel particle. The term "hydrogel" as used herein refers to a water-containing, but water-insoluble polymer network, wherein the polymer chains are linked to a three-dimensional network by chemical bonds or physically, for example by entangling. By incorporated hydrophilic polymer components the hydrogel swells in water under substantial increase in volume, but without losing material cohesion.

The deformable functionalized hydrogel particles should preferably be in the range of 10-50 μm (micrometer)

in size, should obtain a well-defined spherical shape and be highly compliant and swellable in water to form a large contact area between the probe and the sample. Furthermore the hydrogel material of deformable hydrogel particles should be non-charged and have no unspecific interactions towards charged or hydrophobic surfaces, proteins or cells and should be chemically addressable to be target molecule or ligand functionalized. Therefore deformable functionalized hydrogel particles could be made in general of hydrophilic and uncharged polymers, preferably polyether, like polyethylene glycol (PEG), polypropylene glycol, polyamides, for example polyacrylamides, polyoxazolines, polyalcohols, like polyvinyl alcohol, polysaccharides like agarose, or dextran.

One aspect of the invention are methods referring to hydrogel particle having a Young's modulus between 0.1 kPa and 100 kPa, more preferably between 1 kPa and 50 kPa and even more preferred 20 kPa and 40 kPa. Preferred are further methods wherein the hydrogel particles have a diameter between 5 to 100 μm, more preferably between 10 to 50 μm. Therefore the hydrogel particle may also be called microparticle. For determination of the diameter the particles are visualized on a light microscope and the size of particles is determined as the average over 100 particles.

The inventors could show that hydrogel particles based on PEG are especially suitable because of its low unspecific interactions with surfaces. Based on radical surface chemistry using benzophenone as photoinitiator to graft polymerizable monomers to the particles it is able to introduce various functionalities like carboxy or amine groups directly to the PEG network. Thereby, these functional groups are accessible and can be further functionalized which means ligands or target molecules may be coupled to the particles.

Therefore, one preferred embodiment of the present invention relates to a method, wherein the hydrogel particle are made of polyethylene glycol (PEG) which is functionalized with $-CH_2-COOH$, $-CH_2-CO-NH_2$, $-CH_2-CO-NH-CH=CH_2$, $-CH_2-CO-NH-C≡CH$ or

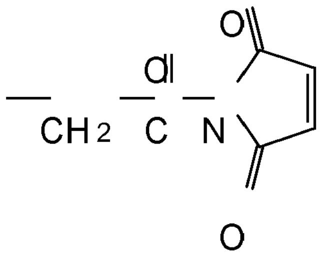

.

One kind of the above-mentioned functional groups is attached in a random manner (randomly distributed) on the carbon atoms of the PEG chain. The PEG consists of 10 - 2000 monomeric units ($-CH_2-CH_2-O$), more preferably of 50 - 500 and even more preferably of 150 - 250 monomeric units.

The functionalized PEG can be represented by the following general formula:

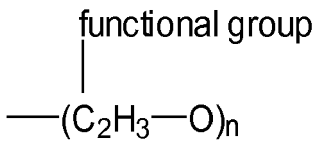

wherein n is an integer from 10 to 2000, more preferably 50 - 500, and even more preferably 150 - 250 and the term "functional group" refers to one of the above-mentioned functional groups which are attached in a random manner to 5% to 30% of the carbon atoms of the PEG chain or in a random manner to 10% to 60% of the ethylenyl groups (-C-C-) of the PEG chain.

It is further preferred that the PEG based hydrogel particles lack hydrogen bond donating groups which reduces unspecific interactions between the hydrogel particles and the surface of the solid carrier. Furthermore, for the PEG-hydrogel particles suitable for the inventive methods preferably long and flexible PEG macromonomers (4 - 20 kDa) are used.

Therefore, one preferred embodiment of the present invention relates to a method, wherein the deformable, target molecule or ligand functionalized hydrogel particles are synthesized using a process comprising the following steps:

i) providing a homogeneous dispersion of poly(ethylene glycol)-diacrylamide, preferably at a concentration of 0.5 wt%
ii) adding a UV photoinitiator, preferably at a concentration of 0.01 wt%
iii) photopolymerizing using a light curing unit

Preferably the above process for synthesizing target molecule or ligand functionalized hydrogel particles used

by the inventive methods comprise further the following steps obtaining carboxylate and amino functionalization:

iv) adding benzophenone preferably at a concentration of 3 wt%
v) adding 2-aminoethyl methacrylate or acrylic acid, preferably at a concentration of 15 vol%
vi) flushing Argon through the suspension
vii) photopolymerizing using a light curing unit

The ligand or target molecules may then added to the hydrogel particles during further functionalization via amide coupling.
This method for producing the hydrogel particles is especially favorable because the modulus of elasticity of the hydrogel particles may conveniently adjusted by the chain length of the polymer and concentration of the UV photoinitiator. Furthermore the modulus may be varied in a range of 1 kPa - 100 kPa, by controlling the degree of swelling of the still not cross linked PEG dispersion by changing the temperature and salt concentration.
Another important advantage of the hydrogel particles synthesized according to this method is that the degree of functionalization (ligand density) can be adjusted easily via the UV exposure time and concentration of the reactants. With the help of this technique in a two-step addition reaction with e.g. 2-aminoethyl methacrylate, or acrylic acid, the PEG network can be functionalized with amine or carboxyl groups. These groups are suitable for direct coupling or further functionalization and subsequently the covalent attachment of any target molecule or ligand to the hydrogel particle. Suitable target molecules or ligands which can be coupled and preferably covalently coupled to the hydrogel particles are biologically active structures, e.g. protein receptors, sugar ligands, peptide ligands, enzymes, substrates, antibodies, antigens, chelators and the like.
Upon contact of a deformable, hydrogel particle on the surface of a transparent glass slide (see Figure 1) they form a distinct contact area if the chosen binding partners (e.g. ligands and receptors) on hydrogel particle and glass slide interact.
This contact area can be determined with an inverted light microscope in reflection interference contrast microscopy (RICM) mode. The mechanical deformation of hydrogel particles as general measurement principle also allows for alternative detection methods to the photometric method explained above: Detection via surface plasmon resonance (SPR), impedance spectroscopy or acoustic methods (QCM) are possible. These detection methods are sensitive towards changes in mass and dielectric constant close a chip surface. The state of mechanical deformation of a hydrogel particle controls these measurement signals and can be detected by the respective methods. For example, strongly adhering hydrogel particles from large contact areas and would thus lead to a larger apparent mass on a QCM chip when compared to weakly adhering particles. SPR and impedance based detection can be enabled via the same principle as shown by the investigation of cell adhesion on ligand surfaces. (Y. Yanase, et al., Biosens. Bioelectron., 2007, 22, 1081-1086) (M. Saitakis, et al., Cell. Mol. Life Sci., 2012, 69, 357-437). In the framework of the hydrogel based screening method, the hydrogel particles adhere on a chip surface in a similar fashion and can be detected by 1) a shift in surface plasmon resonance frequency of a metal (typically gold or silver) coated biochip (P. Pattnaik, Applied Biochemistry and Biotechnology, 2005, 126, 79-92) 2) a shift of acoustic resonance frequency of a biochip (typically quartz crystal resonators (Z. Shen, et al., Analytical Chemistry, 2007, 79, 2312-2319*)* 3) a shift in electric permittivity of the chip surface as measured by impedance methods (J. S. Daniels, et al., Electroanalysis, 2007, 19, 1239-1257*).* The particles can therefore be directly applied as analytes or markers in standard SPR, impedance and QCM screening devices. Therefore, the measurement signal for these methods is not the actual contact area of the hydrogel particles as in optical detection as described above. Rather, the physical properties of the biochip, plasmon resonance (SPR), mechanical oscillation (QCM) and electric permittivity (impedance) reflect the state of mechanical deformation of the particles on the biochip surface. Optical detection is preferred due to cost efficiency and robustness. However for some analytic problems an optically transparent chip surface may be inadequate therefore these alternative detection methods would be required.
The principle of the method according to the invention is based on a solid carrier with immobilized ligand on its surface (a biochip) and secondly on deformable, target molecule functionalized hydrogel particles. This configuration may also be converted so that the solid carrier has a target molecule immobilized on its surface and the hydrogel particles have been functionalized with a ligand.
Step b) of the method according to the invention refers to solid carrier with immobilized ligand or target molecule on its surface. It is thereby preferred that the ligand or target molecule is attached to the solid carrier by covalent bonding. One particularly preferred embodiment of the present invention is a method comprising step b) providing a solid carrier with ligand immobilized by direct or indirect covalent bonding. Thereby direct covalent bonding is especially preferred.
The surface of the solid carrier should be optically transparent, should have a higher refractive index than water, and should have further a low roughness, preferably <10 nm RMS (root-mean-squared roughness). Another point is that the surface has to be suitable for functionalization with biomolecules, serving as ligands and target molecules.
It is particularly preferred that the carrier is a glass slide or a microtitre plate. A microtitre plate or microplate or microwell plate, is a flat plate with multiple "wells" used as small test tubes. Typically a microtitre plate having 6, 24,

96, 384 or even 1536 sample wells can be used. Microplates are produced from many different materials, like polycarbonate for microtitre plate used for PCR. The most common is polystyrene as used for most optical detection microplates.

"Direct covalent bonding" as used herein refers to immobilization of a ligand or target molecule by reaction of a functional group of the ligand or target molecule with a functional group of the material the carrier is made from, respectively of the hydrogel particles. Possible reactive, functional groups of the carrier may be: phenyl, amino groups, hydroxyl groups, thiols, carbonyls, carboxyls, vinyls, halides such as fluorides, chlorides, bromides and iodides, maleimides; succinimide esters.

"Indirect covalent bonding" as used herein refers to immobilization of a ligand or target molecule on a carrier, and respectively a hydrogel particle, wherein the ligand or target molecule is covalently linked to a second compound which mediates the immobilization to the carrier. It is important that the second compound itself is most probably not bound by any ligand or target molecule used in the inventive method. Further the second compound should be able to be immobilized on the carrier, by covalent or non-covalent bonding. Possible non-covalent interactions are: hydrogen bonds, ionic bonds, van der Waals forces, and hydrophobic interactions. Many polymers, such as polystyrene and polypropylene are hydrophobic in nature.

However, immobilization, especially using indirect covalent bonding, may also occur by strong adhesion. Thus, an effective functionalizing according to the present invention, especially of the solid carrier, may be realized not only by chemical bonding, but by physisorption. As key feature for physisorption acts the phenomenon that the force for adhesion is caused by van der Waals force. Chemisorption as functionalizing form according to the present invention uses chemical bonds between carrier and a ligand or target molecule. Such bond may be covalent, but may also be ionic.

In a preferred embodiment of the present invention functionalizing of a carrier or the hydrogel particles is realized by direct covalent bonding namely a chemical reaction between the carrier or the hydrogel particles and the ligands or target molecules respectively, preferably by a substitution reaction. In a more preferred embodiment of the present invention the carrier and/or the hydrogel particles are modified with a functional group which is capable of leaving the carrier upon reaction with the ligand or target molecule. Such functional group may be bound directly to a composing molecule of the carrier or may be bound to a linker which is directly bound to the composing molecule of the carrier. Thus, in a more preferred embodiment of the present invention the carrier and/or the hydrogel particle is modified to bearing a suitable leaving group. Suitable leaving groups may be halides such as chlorides, bromides and iodides; hydroxides, oximes, maleimides, succinimide, alcohols and esters. Such leaving group may be or may be incorporated in maleimide; α-iodoacetyl; α-bromoacetyl; N-hydroxysuccinimide ester (NHS) and 2-pyridyldithiols. In yet more preferred embodiment the leaving group on the carrier and/or the hydrogel particle is capable of reacting with amines, alcohols and thiols, preferably upon proton exchange.

Modification of the carrier and/or the deformable, functionalized hydrogel particle e.g. by introduction of a suitable leaving group is preferably carried out by reaction of an unmodified carrier and/or the hydrogel particle with a reactive bifunctional molecule, preferably a bifunctional molecule with a molecular bridge or spacer arm between the two functional groups. In a preferred embodiment of the present invention functional groups willingly reacting with the carrier and/or the hydrogel particle comprise sulfosuccinimide esters and succinimides.

Suitable reactive bifunctional molecules for modification of the carrier and/or the hydrogel particle comprise N-(γ-maleimidobutyryloxy) sulfosuccinimide ester (sulfo-GMBO), succinimidyl (4-iodoacetyl) aminobenzoate (sulfo-SIAN), succinimidyl-3-(bromoacetamido)propionate (SBAP), disuccinimidyl glutarat (DSG), 2-pyridyldithiol-tetraoxatetradecane-N-hydroxysuccinimide (PEG-4-SPDP).

There are also carriers commercially available made from polymers with reactive functional groups introduced for covalent bonding. One example are microplates named CovaLink™ NH by Thermo scientific which allow covalent binding through a secondary amine group.

Further it is preferred that the solid carrier is selected from the group comprising or consisting of: a glass slide, polymeric slide, a cover slip, a microtitre plate, cuvette, micro array slides, test tubes, and microfluidic chambers.

During step d) of the inventive method the deformable, target molecule functionalized hydrogel particle and the solid carrier with immobilized ligand forms a complex by binding of the target molecule and the ligand. Subsequently, the target molecule functionalized hydrogel particle come in contact to the ligand on the surface of the solid carrier. Interaction or binding of the ligand to the target molecule leads to adhesion of the deformable hydrogel particle and it comes to mechanical deformation of the hydrogel particle, in case the analyte is able to bind to the target molecule.

Forming a complex occurs by intermolecular forces, such as ionic bonds, hydrogen bonds and van der Waals forces. The docking has to be reversible. Step d) of the inventive methods could also be defined as adding ligand or target molecule functionalized hydrogel particles to the surface of a solid carrier that is functionalized with the respective binding partner (target molecule or ligand) and incubate, preferably at room temperature. In general the inventive methods may be carried out at a temperature avoiding freezing and denaturation of target molecule and ligand (proteins denaturize due to heat (around 60°C) which allows handling at al common room temperatures). During incubating the functionalized hydrogel particles sediment to the surface of the solid carrier. Upon contact, ligands and target molecules bind, the functionalized hydrogel particles adhere and form a distinct contact area with the surface of the solid carrier (Figure 3b).

Sufficiently deformable hydrogel particles will exhibit large contact areas that can be conveniently detected via reflection interference contrast microscopy (RICM) to determine the surface energy W. The hydrogel particles are sufficiently deformable having the above described Young's modulus.

Thereby it is preferred that the functionalized hydrogel particles are dispersed in buffer or water. Step d) as well as the complete method of the invention could be performed in solution. This means that the hydrogel particles of step a) and the analyte of step c) may be solved eventually in the same solvent or in different solvent. Such a solution could be based on any suitable solvent as well as buffer solutions. It should be avoided that the solvent interferes with the inventive method and that the solvent or buffer denaturizes the conformation of the ligand, the analyte or the target molecule. Which elements interferes depends on the used pair of binding partners; ligand and target molecule. For example autocatalytic cleavage of proteases may be depended on $Ca^{2+}$ concentration in the buffer. Nevertheless it could also be desirable to add such components to the buffer in order to detect their effect on analyte binding. Consequently the measurement would be made with different buffers containing one component in different concentrations and the results were compared. In general suitable are buffered solutions on basis of PBS-buffers (Phosphate buffered saline) as well as Tris- and triethanolamine buffer.

Step e) refers to addition of the analyte. Also the analyte may be in a suitable solvent or buffer. Here the same applies as described above in regard to the functionalized hydrogel particles. For control reasons, whether the method is actually working (in the hand of the actual user, under the conditions in the concrete laboratory) a negative probe and a positive reference probe could used together with the analyte to be tested. This is a standardized procedure with biologically or diagnostic assays. This means that at the same time or immediately one after the other not only the analyte is tested within the inventive method but also a negative control, which can be a blank and/or a sample containing a substance to be known not to bind to the target molecule. Such a blank would comprise most probably the solution or buffer of the analyte with all components except for the analyte. Furthermore, the inverse method (target molecule and ligand functionalizing is inverted) can be measured in parallel.

Depending on concentration and affinity addition of the analyte results in a displacement of the bonds between the ligand (target molecule respectively) on the carrier surface and the target molecule (ligand respectively) on the hydrogel particles. The consequent alteration in the contact surfaces between the hydrogel particles and the carrier surface, i.e. the shift to lower binding energies can be detected which results in quantitative information to the binding affinity of the analyte.

Within the inventive method the analyte and the ligand compete for the binding site at the target molecule. If the analyte is able to specifically bind the target molecule of the inventive method, at least a part of the target molecules releases the ligand and binds to the analyte to be tested. This causes inhibition of the interaction between the target molecule functionalized hydrogel particles and the ligand on the surface of the carrier. Depending on the concentration and the affinity of the analyte, this will result in a distinct decrease of the contact area and surface energy (Figure 7b-d).

Said contact surface and changes thereof may be optically determined using a simple reflection-contrast method. The adhesion energy can be derived from measurements of the contact radius with a known Young's modulus of the deformable, functionalized hydrogel particle. From a simple linear-mechanical model the deformation energy during adhesion results *[*Hertz, H., Ueber die Berührung fester elastischer Körper. Journal für die reine und angewandte Mathematik (Crelles Journal) 1882, 1882, (92), 156-171.*]*. The adhesion energy can be directly calculated from the deformation energy (using JKR model - Johnson, Kendall, Roberts - 1964-1971)) and thus the underlying bond energy of the ligand - target molecule bond.

Detection of the contact areas between the hydrogel particles and the surface of the carrier via reflection interference contrast microscopy (RICM) allows determining the surface energy *W*,

$$a^3 = 6\pi \frac{W}{E_{eff}} R^2, \qquad (1)$$

wherein a is the radius of contact area, R radius of the used hydrogel particle and $E_{eff}=[4E/3(1-v^2)]$ its effective elastic modulus, where $v$ is the Poisson ratio and $E$ the elastic modulus (also called Young's modulus). The measurement of the surface energies corresponds to a direct binding assay. This yields the ligand-receptor binding energy, if the surface densities of the binding partners are known, which would require additional experimental data.

Due to the specificity of the interaction and the sensitive response to the concentration of added analytes the measurement principle of the inventive method can be used to perform inhibition competition assays, wherein variation of the analyte concentration and measurement of the respective surface energies yield the half-maximal inhibitory concentration ($IC_{50}$) of the analyte as a measure of the analyte affinity

Thereby inhibition/competition assays according to the invention give straightforward access to the affinities of added analytes, i.e. molecules that bind to either target molecules or ligands. When such analytes are added they

compete with the bonds that were formed, thereby inhibiting the interaction between the deformable hydrogel particles and the surface of the solid carrier. Depending on the concentration and the affinity of the analyte, this will result in a distinct decrease of the contact area and surface energy (Figure 3b-d). If all binding sites are occupied, the hydrogel particles detaches from the surface of the carrier. Consequently, detection of the changes in contact area upon analyte addition yields the inhibitory concentration and thus the binding affinity of the analyte.

Therefore one preferred embodiment of the invention refers to a method, wherein step f) determining the alteration of mechanical deformation of the hydrogel particles of step a) allows for calculating the binding affinity and/or the concentration of the analyte.

Step f) of the inventive methods comprises preferably determining the alteration of mechanical deformation of functionalized hydrogel particles upon binding of the analyte to the target molecule. It is common knowledge that determining an alteration is only possible if a blank sample and/or a sample containing a substance to be known not to bind are also measured, or alternatively determining the mechanical deformation before and after addition of the analyte.

Therefore determining the alteration of mechanical deformation of functionalized hydrogel particles upon binding of the analyte to the target molecule may comprise measuring the contact radius of the deformable, receptor functionalized hydrogel particle bound to the solid carrier with immobilized ligand or respectively the contact radius of the deformable, ligand functionalized hydrogel particle bound to the solid carrier with immobilized target molecule before and after addition of the analyte. Thereby decrease in contact radius indicates binding of the analyte to the target molecule, like a receptor. Therefore the present invention comprises also a method for the detection and/or characterization of an analyte, comprising or consisting of the following steps:

a) providing deformable, target molecule functionalized hydrogel particle
b) providing a solid carrier with immobilized ligand on its surface
c) providing an analyte to be tested
d) forming a complex between the deformable, target molecule functionalized hydrogel particle and the solid carrier with immobilized ligand by binding of the receptor and the ligand
d’) determining the mechanical deformation of hydrogel particles of step a).
e) addition of the analyte and
f) determining the alteration of mechanical deformation of hydrogel particles of step a) in case the analyte binds to the target molecule.

Further embodiment of the present invention relates to a method for the detection and/or characterization of an analyte, comprising or consisting of the following steps:

a) providing deformable, ligand functionalized hydrogel particle
b) providing a solid carrier with immobilized target molecule on its surface
c) providing an analyte to be tested
d) forming a complex between the deformable, target molecule functionalized hydrogel particle and the solid carrier with immobilized ligand by binding of the receptor and the ligand
d’) determining the mechanical deformation of hydrogel particles of step a).
e) addition of the analyte and
f) determining the alteration of mechanical deformation of hydrogel particles of step a) in case the analyte binds to the target molecule.

One preferred embodiment of the present invention refers to a method, wherein step f) comprises optical determining the contact radius of the deformable, receptor functionalized hydrogel particle bound to the solid carrier with immobilized ligand and is preferably carried out using reflection interference contrast microscopy (RICM).

The measured change in mechanical deformation or in resulting changes in contact surfaces can be considered as analogous to the change in the resonant frequency at a SPR chip. However, in contrast to this method alteration of the deformation of the hydrogel particles and hence of the contact area serves as a direct, optically read out with the aid of a microscope or a camera.

This measurement principle has the following advantages compared to the prior art:

Compared to other label-free methods: (e.g. SRP, QCM)

- less expensive consumables; e.g. does not need special carriers like gold vaporized (SRP) or quarz (QCM) surfaces
- suitable for miniaturizing and automatization
- no technical limitation in sense of medical-diagnostic samples (complex cell culture media or blood serum)

- no active temperature control is required
- optical detection method
- solid carrier, or biochips may easily be regenerated
- advantages in regard to sensitivity and selectivity especially within low molecular weight analytes or analytes having a refraction index near water, or having low affinity to the target molecule.

Compared to methods using labels (ELISA):

- quantitative results for concentration is possible even without the use of a standard curve
- binding affinity may be analyzed
- real time measurement allows study of binding kinetics
- effect of small molecules on binding, like Ions, may be tested
- no cross reactivity or false positive results caused by the label
- no time consumable washing and blocking steps necessary

Biochip-based label-free methods for detection of analytes like the inventive methods show significantly improved measurement properties, because they allow a quantitative determination of the analyte, real-time measurement and avoid the use of markers that cause cross-reactions and potentially lead to false diagnoses. Existing label-free method are not really suitable for use in medical diagnosis because they are too expensive and require laboratory conditions. In contrast the methods of the present application are very well suitable for use in medical diagnosis because they are cheap and robust and have higher selectivity and sensitivity compared to label-free methods of the prior art especially in regard to low molecular weight analytes. The hydrogel particles can easily chemically modified and thus represent a simple platform to be adapted for the establishment of new or modified assays.

Hence one embodiment of the present invention refers to the use of the inventive methods in medical diagnosis.

The analysis and characterization of individual analytes, based on the analysis of the contact area of individual hydrogel particles, is of interest for basic research, however, it is time consuming and therefore less suitable for the screening for new drug candidates.

Therefore the present invention refers also to methods for detecting and characterizing an analyte based on a novel optical method to determine the total contact area (total deformation) of an array composed of many tightly packed hydrogel particles (see Figure 2). Such an array consists of a large amount of target molecule functionalized hydrogel particles within a limited space, like a well of a microtiter plate, with a ligand functionalized surface (a cover slip on the bottom of the well or the surface of the bottom of the well).

The inventors could show that change of the totality of contact area in an array upon the addition of an analyte causes a change in brightness reflection contrast image. This change in brightness can be read photometrically and allows direct conclusions about the change in the contact surfaces of the target-molecule or ligand functionalized hydrogel particles without image processing steps on individual contact surfaces. The determination of measurable values, such as $IC_{50}$ values of an analyte is possible by changing the concentration of the analyte and therefore detection of different levels of brightness. Hence the inventive method is suitable for automation, higher throughput (using microtiter plates). This allows an improved manageability, and reduces costs compared to methods based on alternative detection methods. Furthermore the photometric detection within the inventive method allows a high time resolution, and thus determination of the binding or detachment kinetics of the analytes to the target molecules is possible. Therefore one preferred embodiment of the presdent invention refers to methods wherein step f) of the inventive methods comprises photometrically determining a change in brightness.

In regard to this alternative of step f) the use of cheaper low-resolution lenses (with $\lambda$/4-attachment) instead of high-resolution object lens is possible and allows detecting arrays with a size of several millimeters. The measuring signal is no longer detected by digital image, but directly with the aid of a photometer coupled to the microscope. Further requirements are a constant monochromatic illumination (LED) and high gray scale and brightness resolution, which are realizable in nowadays in a simple setup. This inventive, simplified detection technology allows an automated screening of many analytes on e.g. microtiter plates (Figure 2) or stopped-flow cells (Figure 4).

The inventive method can also be used as a cost and label-free alternative to conventional screening methods in drug development or basic biological and biochemistry research. The setup consists of glass micro titer plates (e.g. mikroglas chemtech GmbH), which, in principle, are multiple biochips or other solid carriers as defined herein. Individual wells of the micro titer plate may be functionalized using silane surface chemistry with ligands or target molecules respectively. Then deformable functionalized hydrogel particle functionalized hydrogel particles are added which sediment on the solid carrier surface forming an array arrangement. The pair of ligand and target molecule conjugated to the carrier (biochip) and the hydrogel particle depends on the analyte to be detected. For example, using the inventive method multiple or specific kinase inhibitors as analytes can be detected being important for current drug discovery. A kinase assay may composed as follows: The kinase (for example protein kinase G) is covalently bonded to a glass-titer

plate (carrier) and hydrogel particles are functionalized with a kinase inhibitor peptide (Arg-Lys-Arg-Ala-Lys-Glu) knowing to bind to the kinase. Therefore the hydrogel particles functionalized with the kinase inhibitor peptide bind to the kinase on the glass-titer plate. After addition of various potential kinase inhibitors (drug candidates) as analytes, the resulting change in the radius of contact surfaces upon binding of an analyte is read photometrically. This gives direct conclusions about the binding affinity of the analyte. This method allows a real high-throughput screening assay.

Therefore, one embodiment of the present invention is a high throughput screening method for the detection and/or characterization of an analyte based on optical determining the alteration of mechanical deformation of hydrogel particles, preferably forces based mechanical deformation of hydrogel particles.

One preferred embodiment of the inventive method is designed in a way that the analyte binds (in case the analyte is able to bind to the target molecule) the functionalized hydrogel particle but not to the ligand immobilized on the solid carrier this causes that regeneration of the solid carrier can be avoided entirely.

The analytes binding to the deformable hydrogel particles alter the measured signal but do not interact directly with the solid carrier. By their relatively large volume (large hydrodynamic resistance) the hydrogel particles with the bound analyte can be easily removed from the surface of the carrier. By removing them one obtains a completely regenerated surface of the carrier. The purification alone with hydrodynamic means (i.e. flushing) is not possible in regard to the most label-free methods of prior art because small molecules bind to the chip surfaces that have no hydrodynamic resistance.

The ligand has to be choosen to bind to the same site (binding pocket, epitope) of the target molecule as the potential analyte or at least bind in a way that its interaction to the target molecule is affected by the analyte. For the inhibition/competition assay the analyte and the ligand has to compete for binding to the target molecule.

The term ligand as used herein refers to a substance which specifically binds or ligates to the target molecule. The ligand-specific binding reagent is therefore the target molecule. One preferred embodiment of the present invention refers to a method, wherein the ligand is chosen from the group comprising or consisting of: ligand of a receptor, substrate, antigen, signaling molecule, subunit of a protein complex, neurotransmitter, pheromone, toxin, toxoid, drug, second messenger, transcription factor, and metabolite.

The term "ligand of a receptor" as used herein refers to a signal triggering molecule, binding to a site on a target protein. The term "substrate" as used herein refers to substances upon which an enzyme acts in a biochemical reaction. The term "antigen" as used herein refers to a substance that evokes the production of one or more antibodies by a human or animal. Binding between antibody and antigen is based on an interaction similar to the fit between a lock and a key. The substance may be from the external environment or formed within the body. The term "signaling molecule" as used herein refers to a chemical substance released from a cell and is involved in transmitting information between cells. The term "neurotransmitters" as used herein refers to endogenous substances that transmit signals from a neuron to a target cell across a synapse. The term "pheromone" is a secreted or excreted substance that triggers a social response in members of the same species. Pheromones are thereby chemicals acting outside the body. The term "subunit of a protein complex" refers to a protein which may be part of an assembly of more than one polypeptide chain wherein all assemblies of proteins are considered as protein complexes, also complexes of proteins involving RNA molecules, like ribosome. A toxin as used herein is a small molecule, peptide, or protein that is capable of causing disease on contact with or absorption by body tissues produced within living cells or organisms. A toxoid is a weakened or suppressed toxin. The term "drug" as used herein refers to a substance which has medicinal, intoxicating, performance enhancing or other effects when administered to a human animal body and is not considered a food or exclusively a food. The term "second messenger" as used herein refers to a molecule that relays signals from the cell surface to target molecules inside the cell, thereby normally amplifying the strength of the signal. The term "transcription factor" or "sequence-specific DNA-binding factor" as used herein refers to each protein that binds, directly or indirectly, to specific DNA sequences, thereby controlling (activating or blocking) transcription of genetic information from DNA to mRNA. The term "metabolites" as used herein refers to intermediates and products of metabolism of a human or animal body.

Another preferred embodiment of the present invention refers to a method, wherein the target molecule is chosen from the group comprising or consisting of: receptors, antibodies, enzymes, subunits of a protein complex, response elements (DNA), ligands, chelators, and peptides.

The term "receptor" as used herein refers to a structure on the surface of a cell or inside a cell that selectively receives chemical signals originating externally from the cell and directs a cell to do something.

The term "antibody" or alternatively "immunoglobulin (Ig)" as used herein refers to a protein of the immune system, produced normally by B cells, that circulates in blood and lymph in response to an antigen. Antibodies bind their respective antigens through complementarity regions which are highly variable.

Generally, all current types of antibodies may be used, such as for example polyclonal antibodies, monoclonal antibodies, humanized antibodies, human antibodies, chimeric antibodies, recombinant antibodies, bispecific antibodies as well as antibody fragments, wherein monoclonal antibodies are preferred.

Antibody fragments, aptamers, mimotopes, fab fragments, fc fragments, peptides, lectins, nucleotides, peptidomimetics, gapmers, ribozymes, CpG-oligomers, DNAzymes, riboswitches or lipids may be used instead of antibodies.

The term "enzyme" as used herein refers to a substance which is able to catalyze one or more biochemically reactions. Enzymes are mainly proteins but also some RNA molecules, like snRNA may act as an enzyme. The term "response element" as used herein refers to short nucleotide sequences within a promoter of the DNA, which are recognized as a binding site for transcription factors. The term "extracellular matrix" as used herein refers to components of the extracellular part of animal tissues that usually provides structural support. Nevertheless other functions are regulating intercellular communication and cell's dynamic behavior, which is based on specific interactions and binding to cells, enzymes or among each other.

Methods for producing said target molecules but also the ligands, especially antibodies and monoclonal antibodies, are known to the one skilled in the art.

The analyte or the substance to be tested for binding may be each chemical molecule. Any analyte against which antibodies can be produced or which may be bound to a known target molecule can be used as substance to be detected. The present invention therefore comprises methods, wherein the analyte is chosen from the group comprising or consisting of: antibodies, pharmaceuticals, antibiotics, cytostatics, psychoactive substances, narcotics, analgesics, cardiac drugs, metabolites, coagulation inhibitors, hormones, interleukins, cytokines, performance-enhancing drugs, drugs, toxins, molecules derived from pathogens, viruses, prions, bacteria or parasites, noxious substances, pesticides, insecticides, wood preservatives, herbicides, fungicides, explosives, sugars, vitamins and flavors.

Examples of possible analytes are listed in the following:

Analytes from human diagnostics include:

- Molecules derived from pathogens, viruses, prions, bacteria or parasites,
- Tumor markers and other biological markers
- Antibodies
- Metabolites
- Hormones, like human chorionic gonadotropin (hCG) as indicator for pregnancy
- performance-enhancing drugs

Examples of molecules derived from pathogens, viruses, prions, bacteria or parasites include: toxins, like mycotoxins, bacterial toxins, e.g. cholera toxin, pertussis toxin and specific glycolipids or glycoproteins from pathogens, like hemaglutinin, and RNA or DNA of pathogens.

Examples of tumor markers include: beta 2 microglobulin, ferritin, p53, corticotropin (ACTH), growth hormone (GH, hGH), prolactin (PRL), chorionic gonadotropin (hCG and CG), calcitonin (CT), thyroglobulin (tg), carcinoembryonic antigen (CEA), CA antigens 125, 72-4, 15-3 and 19-9, alpha-fetoprotein (AFP), prostate specific antigen (PSA), galactosyl transferase II, and HER-2/neu.

A biomarker, or biological marker, is an indicator of a biological state. It is a characteristic molecule which's concentration or binding activity may be measured and evaluated using the inventive method as an indicator of normal biological processes, pathogenic processes, or pharmacologic responses to a therapeutic intervention. A biomarker may also be a molecule that allows for the detection and isolation of a particular cell type (for example, the protein Oct-4 is used as a biomarker to identify embryonic stem cells) and may be a DNA sequence that causes disease or is associated with susceptibility to disease. Examples of other biological markers are: inflammatory factors, like cytokines, rheumatoid factor, natriuretic peptides (ANP and BNP), plasma proteins and other plasma molecules and homocysteine.

Examples of inflammatory factors are interleukins and cytokines, like interleukin 1 to 18, tumor necrosis factor-$\alpha$, $\beta$ (TNF-$\alpha$, $\beta$), leukotrienes.

Examples of plasma proteins and other plasma molecules include:

C-reactive protein (CRP), albumin, coagulation factors, complement factors, immunoglobulins (IgA, IgE, IgG, IgM), phosoholipases, vasopressin, myoglobin, troponin I, caspases, insulin, blood group determination factor, rhesus factor, alpha amylase, cholinesterase, creatine kinase, gamma-GT, GOT/ ASAT, GPT/ ALAT, LDH, pancreatic lipase, phosphatases, bilirubin, glucose, creatinine, lipoproteins.

Examples of antibodies include antibodies against pathogens as well as antibodies against endogenous structures indicative for autoimmune diseases so called autoantibodies. Autoantibodies are for example Anti-SSA/Ro autoantibodies (systemic lupus erythematosus), anti-La/SS-B autoantibodies (sjögren's syndrome), Anti-dsDNA, anti-cardiolipin antibodies, mitochondrial (MA) ab (primary biliary cirrhosis), thyroglobulin (tg) ab, thyroid peroxidase (TPO, Hashimoto's thyroiditis), glutamic acid decarboxylase (GAD, diabetes mellitus type 1), and anti-transglutaminase antibodies (coeliac disease).

Metabolites are the intermediates and products of metabolism, usually small molecules. Examples of metabolites are: hormones (steroid hormones, gonadotropins, etc.) triiodothyronine, lipids (cholesterol, etc.), glucose, amino

acids, squalene, 5' guanylic acid, antioxidants, like isoasorbic acid, organic acids, like acetic acid, lactic acid, and polyols, like glycerol.

Examples of hormones include:

ACTH, C-peptide, cortisol, DHEA-S, estradiol, estriol, testosterone, insulin, proinsulin, progestrone, PTH.

Examples of performance-enhancing drugs include:

anabolic agents, e.g. bolasterone, boldenone, dehydrochloromethyltestosterone, dihydrotestosterone, clostebol, fluoxymesterone, mesterolone, metandienone, metenolone, methyltestosterone, nandrolone, norethandrolone, oxandrolone, oxymesterone, oxymetholone; stanozolol, testosterone and chemically or pharmacologically related compounds; beta-2 agonists (e.g. clenbuterol); amphetamines: e.g. amineptine, amphetamine, amphetaminil, benzphetamine, dimethylamphetamine, ethylamphetamine, fenetylline, fenproporex, furfenorex, mesocarb, methoxyphenamine, methylamphetamin, methylphenidate, morazone, pemoline, phendimetrazine, pipradol, pyrovalerone and chemically or pharmacologically related compounds; corticosteroids for oral, intramuscular or intravenous administration; peptide hormones and analogs thereof: e.g. chorionic gonadotropin HCG (human sex hormone), corticotrophin (ACTH), growth hormone (HGH, somatotropin), erythropoietin (EPO). Any corresponding factors released from the substances mentioned above are equally forbidden.

Stimulants: e.g. amiphenazole, coffein, cathin, chlorphentermine, clobenzorex, clorprenaline, cropropamide, crotethamide, ephedrine, etafedrine, etamivan, fencamfamin, mefenorex, methylephedrine, nikethamide, pentetrazole, phenylpropanolamine, prolintane, propylhexedrine, strychnine and chemically or pharmacologically related compounds.

Narcotics/ analgesics: e.g. alphaprodine, anileridine, buprenorphine, dextromoramide, dextropropoxyphen, diamorphine, dipipanone, ethoheptazine, ethylmorphine, levorphanol, methadone, morphine, nalbuphine, pentazocine, pethidine, trimeperidine and chemically or pharmacologically related compounds.

Analytes from environmental diagnostics (air, water and soil analyses) are: Noxious substances, like pesticides (PCB, atrazine, etc.), insecticides (pyrethroids, bacillus thuringiensis toxin etc.), wood preservatives (Lindan, etc.), herbicides (diuron, monuron, etc.), fungicid, toxins, explosives (e.g. dioxin, TNT, etc.), colors (e.g. phthalates, azo dyes, glycol ethers, glycol esters, dioxins) and varnishes as well as environmental pollutants.

Examples of insecticides include:

dimethoate, deltamethrin, diflubenzuron, alpha-, zeta-cypermethrin, fenoxycarb, tebufenpyrad, pirimicarb, azadirachtin, piperonyl butoxide, pyrethrine, flocoumafen, chlorpyrifos, permethrin, chalcogran, decadiene carboxylic acid methyl ester, ipsdienol, (S)-cis-verbenol, methylbutenol

Examples of pesticides include:

3,4,5-trimetacarb, 3-hydroxycarbofuran, 5-hydroxy-clethodim sulfone, 5-hydroxy imidacloprid, 5-hydroxy thiabendazole, 6-chloro-3-phenyl-pyridazine-4-(pyridate metabolite), acephate, acetamiprid, acibenzolar-S-methyl, aclonifen, acrinathrin, alachlor, aldicarb, aldicarb sulfoxide, aldoxycarb, ametryn, amidosulfuron, aminocarb, anilazine, atrazine, avermectin B1a, avermectin B1b, azamethiphos, azinphos-ethyl, azinphos-methyl, azoxystrobin, benalaxyl, bendiocarb, benfuracarb, bensulfuron methyl, benzoximate, bifenox, bitertanol, boscalid, bromacil, bromuconazole, bupirimate, buprofezin, butocarboxim, butocarboxim sulfoxide, butoxycarboxim, buturon, carbaryl, carbendazim, carbetamides, carbofuran, carbosulfan, carboxin, carfentrazone-ethyl, chlorbromuron, chlorfenvinphos, chlorfluazuron, chloridazon, chlorotoluron, chloroxuron, chlorpyrifos, chlorsulfuron, chlorthiophos, cinosulfuron, clethodim, clethodim imine sulfone, clethodim imine sulfoxide, clethodim sulfone, clethodim sulfoxide, clodinafop-propargyl, clofentezine, clomazone, clopyralid, cloquintocet-mexyl, coumaphos, cyanazine, cyanofenphos, cyazofamid, cycloate, cymoxanil, cyprodinil, cyromazine, daminozide, deltamethrin, demeton-s-methyl, demeton-s-methyl-sulfone, desmedipham, desmethylformamido-pirimicarb, desmethyl-pirimicarb, dialifos, di-allate, diazinon, dichlofluanid, dichlorvos, diclobutrazol, diethofencarb, difenoconazole, difenoxuron, diflufenican, dimefuron, dimethachlor, dimethenamid, dimethoate, dimethomorph, diniconazole, diphenylamine, disulfoton, diuron, dodemorph, EPN, epoxiconazole, ethiofencarb, ethiofencarb sulfone, ethiofencarb sulfoxide, ethion, ethirimol, ethofumesate, ethoprophos, etofenprox, etrimfos, famoxadone, fenamiphos, fenarimol, fenazaquin, fenbuconazole, fenfuram, fenhexamid, fenoxaprop-ethyl, fenoxycarb, fenpiclonil, fenpropathrin, fenpropidin, fenpropimorph, fenpyroximate, fenthion, fenuron, flamprop-isopropyl, flamprop-methyl, flazasulfuron, florasulam, fluazifop-butyl, flufenacet, flufenoxuron, fluometuron, fluoroglycofene-ethyl, fluquinconazole, fluridone, fluroxypyr-meptyl, flurtamone, flusilazole, flutriafol, folpet, fonofos, fosthiazate, fuberidazole, furathiocarb, haloxyfop-etotyl, haloxyfop-methyl, heptenophos, hexaconazole, hexazinone, hexythiazox, imazalil, imidacloprid, indoxacarb, iodosulfuron-methyl, iprodione, iprovalicarb, isazophos, isofenphos, isoproturon, isoxathion, kresoxim-methyl, linuron, malaoxon, malathion, MCPA-butotyl, mecarbam, mepanipyrim, metalaxyl, metamitron, metazachlor, metconazole, methabenzthiazuron, methamidophos, methfuroxam, methidathion, methiocarb, methomyl, methoxyfenozide, metobromuron, metolachlor, metosulam, metoxuron, metribuzin, metsulfuron-methyl, molinate, monocrotophos, monolinuron, monuron, myclobutanil, naled, napropamides, neburon, nicosulfuron, nicotine, nuarimol, ofurace, omethoate, oxadixyl, oxamyl, oxamyloxime, oxycarboxin, oxydemeton-methyl, paclobutrazol, paraoxon-methyl, parathion, parathion-methyl, penconazole, pencycuron, pendimethalin, phenmedipham, phenthoate, phorate, phorate sulfoxide, phosalone, phosmet, phosphamidon, phoxim, picolinafen, picoxystrobin, pirimicarb, pirimiphos-ethyl, pirimiphos-methyl, primisulfuron-methyl, prochloraz, procymidone, profenofos, promecarb, prometryne, propachlor, propamocarb, propargite, propetamphos, propham, propiconazole, propoxur, propyzamide, prosulfocarb, prosulfuron, prothiofos, pymetrozine, pyraclostrobin, pyrazophos, pyridaben, pyridaphenthion, pyridate, pyrimethanil, pyriproxyfen, quinalphos, quinmerac, quinoclamine, quinoxyfen, quizalofop-ethyl, rimsulfuron, rotenone, simazine, simetryn, spiroxamine, sulfosulfuron, sulfotep, sulprofos, tebuconazole, tebufenozide, tebufenpyrad, tebutam, tebuthiuron, terbacil, terbufos, terbumeton, terbuthylazine, terbutryn, tetrachlorvinphos, tetraconazole, thiabendazole, thiacloprid, thiamethoxam, thifensulfuron-methyl, thiodicarb, thiofanox, thiofanox sulfone, thiofanox sulfoxide, thiophanate (-ethyl), thiophanate-methyl, tolclofos-methyl, tolylfluanid, triadimefon, triadimenol, triasulfuron, triazophos, tribenuron-methyl, trichlorfon, tricyclazole, trietazine, trifloxystrobin, triflumizole, triflusulfuron-methyl, triticonazole, vamidothion.

Examples of noxious substances and toxic substances include:

1,1,1-trichloroethane, 1,1,2,2-tetrachloroethane, 1,1,2-trichloroethane, 1,1-dichloroethane, 1,1-dichloroethene, 1,2,3,4,6,7,8,9-octachlorodibenzofuran, 1,2,3,4,6,7,8-heptachlorodibenzo-p-dioxin, 1,2,3-trichlorobenzene, 1,2,3-trichloropropane, 1,2,4-trichlorobenzene, 1,2-dibromo-3-chloropropane, 1,2-dibromoethane, 1,2-dichlorobenzene, 1,2-dichloroethane, trans-1,2-dichloroethene, 1,2-dichloroethylene, 1,2-diphenylhydrazine, 1,3,5-trinitrobenzene, 1,3-butadiene, 1,3-dichlorobenzene, cis- and trans-3-dichloropropene, 1,4-dichlorobenzene, 2,3,4,7,8-pentachlorodibenzofuran, 2,3,5,6-tetrachlorophenol, 2,3,7,8-tetrachlorodibenzofuran, 2,3,7,8-tetrachlorodibenzo-p-dioxin, 2,4,5-trichlorophenol, 2,4,6-trichlorophenol, 2,4,6-trinitrotoluene, 2,4-dichlorophenol, 2,4-dimethylphenol, 2,4-dinitrophenol, 2,4-dinitrotoluene, 2,6-dinitrotoluene, 2-butanone, 2-chloroaniline, 2-chlorophenol, 2-hexanone, 2-methylnaphthalene, 3,3'-dichlorobenzidine, 4,4'-methylenebis(2-chloroaniline), 4,6-dinitro-o-cresol, 4-aminobiphenyl, 4-nitrophenol, acenaphthene, acetone, acrolein, aldrin, alpha-chlordene, amosite asbestos, aroclor 1016 , 1221, 1232, 1240, 1242, 1248, 1254, 1260, 1262, arsenic acid, asbestos, benzene, benzidine, benzo(a)anthracene, benzo(a)pyrene, benzo(b or k)fluoranthene, benzofluoranthene, benzopyrene, bis(2-chloroethyl) ether, bis(2-ethylhexyl)adipate, bis(2-methoxyethyl) phthalate, bromodichloroethane, bromoform, butyl benzyl phthalate, butylate, calcium arsenate, carbazole, carbon tetrachloride, carbophenothion, chlordane, chlordecone, chlorobenzene, chlorodibromomethane, chloroethane, chloromethane, chlorpyrifos, chrysene, chrysotile asbestos, cis-chlordane, coal tar creosote, para-cresol, cresols, cyanide, cyclotrimethylenetrinitramine (RDX), o,p'-or p,p'- ddd, p,p'-dde, o,p'- or p,p'-ddt, di(2-ethylhexyl)phthalate, diazinon, dibenzo(a,h)anthracene, (chlorinated) dibenzofuran, dibenzothiophene, dibromochloropropane, dichlorobenzene, dichloroethane, dichloroprop, dichlorvos, dicofol, dieldrin, dimethoate, dimethyl formamide, dimethylarsinic acid, di-n-butyl phthalate, dinitrotoluene, disulfoton, (alpha-; beta-) endosulfan (sulfate), endrin (aldehyde; ketone), ethoprop, ethyl ether, ethylbenzene, fluoranthene, formaldehyde, gamma-chlordene, guthion, heptachlor, heptachlor epoxide, heptachlorobiphenyl, heptachlorodibenzofuran, heptachlorodibenzo-p-dioxin, hexachlorobenzene, hexachlorobutadiene, alpha-, beta-, delta-, gamma-hexachlorocyclohexane, hexachlorocyclopentadiene, hexachlorodibenzofuran, hexachlorodibenzo-p-dioxin, hexachloroethane, hydrazine, hydrogen cyanide, indeno(1,2,3-cd)pyrene, methoxychlor, methyl isobutyl ketone, methyl parathion, methylene chloride, methylmercury, naled, naphthalene, n-nitrosodimethylamine, n-nitrosodi-n-propylamine, n-nitrosodiphenylamine, ortho-cresol, oxychlordane, parathion, pentachlorobenzene, pentachlorobiphenyl, pentachlorobutadiene, pentachlorodibenzofuran, pentachlorodibenzo-p-dioxin, pentachlorophenol, phenanthrene, phenol, phorate, polonium-210, polybrominated biphenyls, polychlorinated biphenyls, polycyclic aromatic hydrocarbons, p-xylene, pyrene, pyrethrum, s,s,s-tributyl phosphorotrithioate, strobane, styrene, tetrachlorobiphenyl, tetrachlorodibenzo-p-dioxin, tetrachloroethane, tetrachloroethylene, tetrachlorophenol, thiocyanate, toluene, toxaphene, trans-chlordane, tributyltin, trichlorobenzene, trichloroethane, trichloroethylene, trichlorofluoroethane, trifluralin, vinyl chloride.

Analytes in process engineering/ food technology/ biotechnology/ cosmetics:

chemical and biotechnological synthesis products (inclusively primary products, intermediate products, byproducts and degradation products, such as vitamins, flavors, pharmaceuticals, toxins, lipids, amino acids, glycerides, carbohydrates, fibers, sweeteners, colorants, preservative agents, polysaccharides, mycotoxins, etc.

Examples of food additives include:

acesulfam K, acetylated oxidated starch, acetylated starch, acetylated distarch adipate, acetylated distarch phosphate, allura red AC, alpha tocopherol, aluminum lacquers, aluminum sodium sulfate, amaranth, ammonia caramel coloring, ammonia sulfite caramel coloring, annatto, anthocyans, malic acid, ascorbinic acid, aspartame, azorubine, beetroot red, bentonite, benzoic acid, benzyl alcohol, succinic acid, beta-apo-8'-carotenal, beta-apo-8'-carotinic acid ethyl ester, beta-cyclodextrin, beeswax (white and yellow), biphenyl, bixin, boric acid, brown FK, brown HAT, brilliant blue FCF, brilliant black BN, butadienestyrene copolymer, butylhydroxyanisole (BHA), butylated hydroxytoluoene (BHT), calcium 5'-ribonucleotide, canthaxanthin, capsanthin, capsorubin, carbamide, carboxymethylcellulose, carnauba wax, carotenes (mixed carotenes, beta carotene), carrageenan, cellulose (microcrystalline cellulose, cellulose powder), quinoline yellow, chlorophylls, chlorophyllins, cochenille red A, enzymatically hydrolyzed carboxymethylcellulose, erythrosin, gamma tocopherol, sunset yellow S, gellan, green S, hydroxypropyl cellulose, hydroxypropyl distarch phosphate, hydroxypropylmethyl cellulose, hydroxypropyl starch, indigotin I, invertase, copper-containing complexes of the chlorophylls, copper-containing complexes of the chlorophyllins, curcumin, monostarch phosphate, sodium carboxymethyl cellulose, neohesperidin DC, nisin, norbixin, oxydized starch, patent blue V, pectins (pectin, amidated pectin), phosphatized distarch phosphate, phosphoric acid, polydextrose, polyethylene glycol 6000, polyethylene wax oxidates, polyvinyl esters of the non-branched fatty acids C2 - C18, polyvinylpolypyrrolidone, polyvinylpyrrolidone, riboflavin, riboflavin 5'-phosphate, red 2G, saccharin and Na-, K- and Ca-salts thereof (saccharin, saccharin sodium, saccharin calcium, saccharin potassium), sucrose acetate isobutyrate, sorbitan monolaurate, sorbitan monooleate, sorbitan monopalmitate, sorbitan monostearate, sorbitan tristearate, starch sodium octenylsuccinate, tannin, tartrazine, thaumatin, tragacanth, processed eucheuma seaweed, linked sodium carboxymethylcellulose, xanthan.

Macromolecules to be mentioned include in particular polynucleotides, oligonucleotides, DNA fragments, RNA fragments, proteins, glycoproteins, lipoproteins, polysaccharides, DNA, RNA, mimotopes, fab fragments, fc fragments, peptides, lectins, peptidomimetics, gapmers, ribozymes, CpG oligomers, DNAzymes, riboswitches, polymers, hormones, vitamins, carbohydrates, glycerides or lipids.

The method of the present invention is especially suitable for low-molecular weight analytes, like sugars and analyte having a refraction index near water, or having low affinity to the target molecule. Therefore these analytes are especially preferred.

The following samples may be particularly used as sample containing the analyte: saliva samples, blood samples, serum samples, urine samples, mucus samples, tissue samples, optionally minced, water samples, wastewater samples, samples from chemical production processes, extracted soil samples, food samples, samples from biological and biochemical production processes, fermentation solutions, solution of a concentrated analyte in buffer and the like. Serum samples as well as diluted samples of the aforementioned kind or diluted extracts of, for example, soil samples, mucus samples, fermentation solutions and the like are particularly preferred.

The measurement principle of the inventive method has been tested by studying carbohydrate - protein interactions as they are an important example of ligand/receptor interactions for many biological recognition events such as cell signaling, inflammation and viral infections. In addition, the label-free analysis of carbohydrate interactions still represents an analytical challenge. As reference binding partners for method of the invention we used the interaction between mannose as ligand and the receptor Concanavalin A (ConA) as target molecule.

Inhibition assays were conducted to study carbohydrate-lectin interactions using non-labeled analytes, yielding $IC_{50}$ values that agreed well with those obtained from SPR measurements. Chief requirements for robust and sensitive carbohydrate detection method according to the invention based on the mannose and ConA interactions are reduction of nonspecific surface interactions. The inventors could show that the specificity of the interaction and the sensitive response to the concentration of added analytes the measurement principle of the inventive method can be used to perform competition inhibition assays. This procedure involves variation of the analyte concentration and measurement of the respective surface energies. Generally, these assays yield the half-maximal inhibitory concentration ($IC_{50}$) of the analyte as a measure of the analyte affinity. Moreover, the solid carrier can be easily regenerated by a few washing cycles. Subsequent binding studies on the regenerated surfaces gave reproducible results (see Example 5). The practical detection limit in surface energies within the inventive method is on the order of 0.1 $\mu J/m^2$, most likely exceeding the sensitivity of the Surface Force Apparatus and similar methods.

Overall, the inventive method is highly sensitive and shows reproducible results, without the need of expensive instrumentation or consumables that apply to other label-free techniques, e.g. metal coated biochips or microcantilevers. The method has several useful characteristics especially for the characterization of analytes with low molecular mass and refractive indices close to water. New screening assays targeting different classes of analytes can be established without much effort by easy functionalization of PEG hydrogels and glass surfaces.

One embodiment of the present invention relates to a kit comprising at least a deformable, target molecule

functionalized hydrogel particle and a solid carrier with immobilized ligand. Alternatively, the kit may comprise at least a deformable, ligand functionalized hydrogel particle and a solid carrier with immobilized target molecule.

A kit in molecular biology or in medical diagnostics is a package which includes all necessary ingredients for performing a certain method or singular step. Standard chemicals as present in any standard molecular biology or medical laboratory are normally not included. Nevertheless some of these standard chemicals may be indispensable to carry out the diagnosis or the immobilization properly. It is understood that all ingredients are provided in quantities that allow for a proper execution of the desired reactions for the majority of scientific, diagnostic and industrial applications.

Often, but not always, these ingredients are provided in already prepared solutions ready- or close to ready-for-use. There may be also combinations of different ingredients already added together. A further advantage is that such kits used to be verified. Therefore the operator doesn't have to prove again the viability of the diagnostic method and can save on at least some control experiments. Therefore kits are a very popular tool in laboratories in research, diagnostics and industry.

Such a kit according to the invention shall include at least the following components:

a) a deformable, target molecule functionalized hydrogel particle
b) a solid carrier with immobilized ligand

The following components may also be included in such kits:

c) blocking solution
d) sample buffer
e) reference probe (compound known to bind or not to bind to the target molecule)

A blocking solution should be a solution avoiding unspecific binding of the analyte. A sample buffer may be used to dilute the sample of the patient (blood, serum, urine) so that the concentration of the analyte is in the range which can normally be detected by the test system used.

If the kit shall be allow for the immobilization of a ligand on a solid carrier the kit should include at least:

A) a deformable, target molecule functionalized hydrogel particle
B) a ligand for immobilization

Thereby the carrier may be modified, for example the carrier may be functionalized with a linker molecule as described above.

The following components may also be included in such kits:

C) blocking solution
D) sample buffer
E) reference probe (compound known to bind or not to bind to the target molecule)
F) carrier, functionalized with a reactive group suitable for binding of ligands

**Description of the figures:**

Figure 1 shows contact area measurements of deformable, hydrogel particles via RCIM. The contact area is imaged by a light microscope and shows typical interference pattern.

Figure 2 shows simplified measuring principle based on the detection of change in grey values upon addition of analyte instead of determining individual contact surfaces. This measurement technique is suitable for high-throughput screening using microtiter plates.

Figure 3 shows a) Principal of the method according to the invention: a) the ligand functionalized hydrogel particle comes into contact with a receptor functionalized surface of a solid carrier and b) upon contact with the receptor on the surface ligand/receptor complexes form and induce mechanical deformation of the hydrogel particles. c) Inhibition affinity assays by addition of analytes and detection of alteration in contact area detected via RICM until d) a complete detachment of the hydrogel particles from the surface of the solid carrier is observed. The RICM images show the contact area between a hydrogel particle and the receptor functionalized surface of the solid carrier, where a white circle means no contact and a grey circle show the

contact area.

Figure 4 shows a scheme of the method of the invention in a "stopped flow" measurement cell.

Figure 5 shows a general scheme for functionalization of PEG based deformable hydrogel particles.

Figure 6 shows a scheme for functionalization of PEG based deformable hydrogel particles with ConA as a receptor or mannose as a ligand

Figure 7 shows principle of adhesion measurements of deformable functionalized hydrogel particles on functionalized surfaces of solid carrier with a) hydrogel particles bearing mannose ligands and a ConA functionalized glass surface and with b) ConA attached to the hydrogel particles and a mannose functionalized glass surface. c) The adhesion energy can be calculated from the contact radius which is plotted against the particle radius. The data points were fitted with the JKR equation to obtain the adhesion energy W.

Figure 8 shows $IC_{50}$ curves for various molecules inhibiting the mannose/Concanavalin A interaction. The adhesion energy has been normalized to the energy obtained in the absence of inhibitor.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

## EXAMPLES

### Example 1: Synthesis of poly(ethylene glycol)-diacrylamide particles

Deformable hydrogel particles based on PEG were synthesized by precipitation polymerization. Briefly, 0.5 wt-% PEG-diacrylamide ($M_n$ = 8000 Da) (50 mg) was dispersed in a 1 M sodium sulfate solution (10 mL). The UV photoinitiator Irgacure 2959 was added at a concentration of 0.01 wt-% (1 mg) to the dispersion and vigorously shaken and photopolymerized with UV light. Water was exchanged by ethanol and benzophenone 3 wt-% (250 mg) and acrylic acid 15 vol-% (1.5 mL) were added and the mixture was flushed with argon for 30 s and irradiated with UV light. The resulting hydrogel particles were washed with ethanol 3 times and left again in ethanol (cf. Figure 5).

### Example 2A: Functionalization of the particles of example 1 with mannose

Therefore ethanol was exchanged with DMF through several washing steps and the carboxylate functionalized hydrogel particles (0.05 g) were left in 10 mL of DMF. Benzotriazole-1-yl-oxy-*tris*-pyrrolidinophosphonium hexafluorophosphate (PyBOP 0.728 g, 1.40 mmol), 1-hydroxybenzotriazole (HOBt 0.097 g, 0.70 mmol) and triethylamine (195 μL, 1.40 mmol) were added to activate the carboxylic groups. This suspension was shaken for 15 min at RT, then aminoethyl-linked mannose (0.050 g, 0.12 mmol) was added. The mixture was allowed to react for 3 h at RT. The hydrogel particles were washed 3 times with DMF and 3 times with methanol. Sodium methoxide (0.004 g, 0.08 mmol) were added and reacted for 1 h at RT. Then, the hydrogel particles were centrifuged at 5000 rpm for 10 minutes and washed 3 times with methanol and 3 times with pure water (cf. Scheme of Figure 6).

### Example 2B: Functionalization of the particles of example 1 with ConA

Hydrogel particles of example 1 (see Fig. 6, PEG-AA) were washed with a 0.1 M phosphate buffer pH 6.5.

Activation of the carboxyl groups were carried out by addition of 0.1 M EDC (2.5 mL) and 0.1 M NHS (2.5 mL) in phosphate buffer pH 6.5. The particles were incubated for 4 h at room temperature and excess coupling agent was removed by washing with phosphate buffer pH 6.5. 5 mL of ConA in phosphate buffer pH 6.5 (0.2 mg $mL^{-1}$) was added and reacted for 12 h. The resulting hydrogel particles were centrifuged and washed first with PBST buffer (phosphate buffered saline/Tween 20) pH 7.4 and subsequently with lectin binding buffer (50 mM NaCl, 1 mM $MnCl_2$, 1 mM $CaCl_2$, 10 mM HEPES).

The successful and homogeneous functionalization of the hydrogel particles was proven by confocal microscopy.

200 μL mannose bearing hydrogel particles (example 2A) were added in a safe lock tube (eppendorf, Germany) and centrifuged. The supernatant was decanted and 100 μL lectin binding buffer pH 6 and 10 μL Fluorescein isothiocyanate (FITC) labeled Concanavalin A solution (5 mg $mL^{-1}$) were added to the particles. The tube was coated in aluminum foil and incubated for 1 h. The excess solution was centrifuged and the particles were washed with lectin binding buffer. The resulted hydrogel particles were measured on a confocal microscope (LEICA DM IRBE).

For the mannose functionalized hydrogel particles the degree of functionalization was directly measured via UV titration with toluidine blue O of the unreacted carboxyl groups and compared to the pure carboxylate functionalized particles (PEG-AA). We obtained a functionalization degree of 56 μmol $g^{-1}$ (48 %) compared to particles bearing only carboxyl moieties, and thus circa one mannose function for every two PEG chains. To directly measure the capability of the particles to bind to ConA receptors density of bound ConA was determined after incubating the mannose functionalized hydrogel particles in ConA and subsequent amino acid analysis. The mannose functionalized hydrogel particles bound $1x10^{16}$ ConA/$m^2$, thus suggesting complete coverage of the hydrogel particles with the receptors. The ConA functionalized hydrogel particles, were analyzed by confocal microscopy as well as ATR-FTIR to test the degree of functionalization. ConA functionalized hydrogel particles were labeled with fluorescein isothiocyanate (FITC). Therefore 200 μL of the particle suspension was centrifuged and decanted. Than 1 mL of FITC (0.1 mg $ml^{-1}$) in a $NaH_2PO_4$ buffer (10 mM, pH 9) was added, covered with aluminium foil and reacted for 12 h. The excess solution was centrifuged and the particles were washed with MilliQ water. The resulting particles were measured on a confocal microscope. The density of ConA on the particles was $1x10^{16}$ ConA/$m^2$ as determined by amino acid analysis and optical microscopy.

**Determination of Young's modulus of the particles of example 2**

Force-deformation curves for mannose functionalized hydrogel particles of example 2A were measured. The curves were analyzed to obtain the elastic modulus *E* using the Hertz model $(F=4/3 \cdot R^{(1/2)} \cdot E \cdot D^{(3/2)}$ / $(1-V^2)$, with *F* the force, *D* the deformation, *v* the Poisson ratio and R the effective radius of the hydrogel particle and the colloidal AFM (Atomic force microscopes) probe. The resulting Hertz fits with *E* as free parameter are represented as dashed lines. The mannose functionalized hydrogel particles are very well represented by the Hertz theory and the average elastic modulus for these particles was 32±5kPa.

**Example 3: Preparation of functionalized solid carrier**

Coverslips made of glass (∅ 24 mm) were used as a solid carrier (Thermo scientific, Germany) and cleaned prior to use by washing with isopropanol and piranha solution (96% $H_2SO_4$ and 30% $H_2O_2$, 3:1). The coverslips were rinsed with MilliQ water and dried in a nitrogen stream. Amine surfaces were prepared via chemical vapor deposition. Here, the coverslips and 3-aminopropyltriethoxysilane (APTES) (50 μL) on a freshly cleaned coverslip were placed in a desiccator and vacuum was applied for 1 min. The desiccator was sealed and the coverslips were left for 1 h to react with the vapor. The coverslips were rinsed with MilliQ water and dried with nitrogen. Then, the coverslips were placed in PBS buffer pH 7.4 containing 2.5 % glutaraldehyde for 30 min followed by washing with MilliQ water and drying. ConA (0.2 mg $mL^{-1}$) in PBS buffer pH 7.4 was placed on the aldehyde functionalized surfaces for 1 h and prior to the measurements washed with lectin binding buffer.

Mannose surfaces were prepared by preparing first epoxy functionalized coverslips. Therefore, the Piranha cleaned coverslips were placed in a solution of ethanol (114 mL), MilliQ water (6 mL), acetic acid (120 μL) and 3-glycidoxypropyltrimethoxysilane (GLYMO) (3 mL) for 2 h. Additionally, the coverslips were washed with isopropanol and dried under a $N_2$-stream and placed in an oven for 12 h at 150°C. A solution of $\alpha$-thioethyl-mannose (1 mg $mL^{-1}$) in PBS buffer pH 7.4 was placed on the slides for 12 h. The mannose surfaces were washed with PBST buffer pH 7.4 three times and subsequently with lectin binding buffer.

**Example 4: Characterization of Neoglycopolymers using ConA as target molecule and mannose as a ligand**

Firstly, two types of hydrogel particles were prepared: a) hydrogel particles functionalized or conjugated with mannose ligands and b) hydrogel particles functionalized or conjugated with ConA receptors (Fig. 7a, 4b) both according

to example 2.

Furthermore two types of solid carriers were prepared via aminosilan/glutaraldehyde chemistry: a) coverslips with immobilized ConA b) coverslips with immobilized mannose. The surface density of the immobilized ConA and ConA incubated mannose almost reached close packing ($5.3x10^{15}$ ConA per $m^2$), as measured by amino acid analysis. Amino acid analysis gave the total number of amino acids, 7.4 nano mol, on a circular ConA coated glass slide with a diameter of 20 mm. The tetrameric ConA possesses 689 amino acids in total, therefore the amount of bound protein on the slide is 7.4 nano mol / 689 = 11 pico mol ConA receptors, thus 6.6 x $10^{12}$ ConA molecules on the glass slide. As a result the ConA density was 5.3 x $10^{15}$ ConA/$m^2$. Since ConA exhibits an hydrodynamic radius us 4.4 nm at the glass sides coating conditions (pH7.4) the theoretical upper limit of ConA receptors on a flat substrate is 1 x $10^{16}$ ConA/$m^2$. Therefore, the surface bound ConA receptors almost reached close packing in our experiments.

With this setup direct binding assays were performed with varied position of ligands and receptors a) mannose functionalized hydrogel particles and ConA immobilized on glass surface b) ConA functionalized hydrogel particles and mannose immobilized on glass surface (Fig. 7). The surface energies of the functionalized hydrogel particles were obtained via RICM by an inverted microscope (Olympus IX71, Germany) was used to obtain the contact area between the functionalized hydrogel particles and the surface of the solid carrier (of example 3). For illumination a Hg-vapor lamp was used with a green monochromator (546 nm). A Zeiss Antiflex 63 x NO 1.25 oil-immersion objective, additional polarizers to avoid internal reflections and a Zeiss AxiocamHRm camera were used to image the RICM patterns. To conduct the JKR measurements of the adhesion energies, both the contact radius and the particle radius were measured. Image processing and data analysis were done using the image analysis software Image-J (public domain NIH) and the mathematical software OriginPro (OriginLab, USA).

The data was then plotted and fitted with equation 1 (Fig 7d) yielding the surface energy *W* of the hydrogel particles as fit parameter. As mechanical parameters this procedure requires the elastic modulus of the mannose functionalized hydrogel particles and ConA functionalized hydrogel particles (see example 2) and the Poisson ratio (v = 0.5, justified for an isotropic gel).

The surface energy obtained in direct binding for the mannose functionalized hydrogel particles adhered to the solid carrier with immobilized ConA was $25 \pm 2$ $\mu J\ m^{-2}$ and $21 \pm 2$ $\mu J\ m^{-2}$ for the ConA functionalized hydrogel particles adhered to a solid carrier with immobilized mannose. Both systems show similar surface energies indicating similar densities of the binding partners as also shown by amino acid analysis.

The theoretical upper limit of the surface energy can be calculated by considering per mannose 5.2 kcal/mol contribution to the surface energy and assuming that every carrier-bound mannose ligand binds to the two ConA binding pockets on the opposite surface. The other two binding sites of the tetrameric ConA molecules are blocked due to the glass of the carrier and hydrogel particle surfaces, respectively.

The specificity of the functionalized hydrogel particles binding was tested by addition of competitively binding sugar analytes into the measurement cell, e.g. 10 mM $\alpha$-methyl D-mannose. At a critical level of competitive analytes the contact areas of functionalized hydrogel particles and surface energies completely vanish, thus indicating the specificity of the adhesion between the target molecule functionalized hydrogel particle and the solid carrier with immobilized ligand. Importantly, when adding $\alpha$-methyl D-mannose at lower concentrations up to 5 mM the functionalized hydrogel particles are still in contact with the surface of the solid carrier (Fig 7d, cross markers) and surface energy gradually decreases with the concentration of added analyte (Fig 8.) Therefore, depending on their concentration and affinity the ConA receptors are inhibited only partially by the added sugar analytes. This can be directly visualized by the decreasing contact areas upon analyte addition and quantified in terms of surface energy via JKR approach.

Next inhibition competition assays were performed. This procedure involves variation of the analyte concentration and measurement of the respective surface energies. Generally, these assays yield the half-maximal inhibitory concentration ($IC_{50}$) of the analyte as a measure of the analyte affinity.

As analytes we used various monosaccharides: $\alpha$-methyl- D-mannose, D-mannose and D-glucose with different binding affinity to ConA. Further, mono- and multivalent glycopolymers (PAA) with a fixed length of the backbone and increasing number of mannose residues from one to three attached to the polymer were used as analytes. All measurements were conducted with the inventive method shown in Fig 7a, using a mannose functionalized hydrogel particles and ConA immobilized on the solid carrier. With the direct binding between mannose functionalized hydrogel particle and ConA on the carrier surface as a reference, the different analytes were added and for each concentration the decrease in surface energy was determined. Figure 8 shows the surface energies normalized direct binding experiment as a function of the inhibitor concentrations.

The normalized surface energy data showed a characteristic sigmoidal shape that was fitted with the Hill equation to obtain the $IC_{50}$ values (Table 1). Clearly, all carbohydrate inhibitors reduced the surface energy of the mannose functionalized hydrogel particles (Table 1). As expected, for the monosaccharides we observed decreasing affinity from $\alpha$-methyl- D-mannose, D-mannose to D-glucose. With the exception of D-glucose our data is in good agreement with calorimetric studies on the inhibition of ConA in solution. The observed deviations are most likely due to surface binding of the ConA receptors and mannose ligands in the inventive method.

It is well known that multivalent ligands exhibit larger affinities because binding is entropically enhanced. Additionally the ligand scaffold can play an important role on receptor inhibition, e.g. by release of organized water or by steric inhibition due to screening of the receptor in case of large scaffolds. Here we analyzed polymers based on poly(amidoamine) (PAA) substructures with a molecular weight of 3 kDa and different amount of mannose ligands from one mannose moiety up to three on a single chain (PAA-Man1, PAA-Man2, PAA-Man3). These structures show a strong inhibition effect on ConA surfaces with $IC_{50}$ values in the micromolar range. Already the monovalent PAA Man1 showed a two orders of magnitude smaller $IC_{50}$ as compared to $\alpha$-methyl-D-mannose.

The $IC_{50}$ values obtained according to the invention are in good agreement with the values obtained by SPR inhibition assays reported earlier (Table 1). Generally, the method is robust and delivered reproducible $IC_{50}$ values also when comparing the inverted (Fig.7b) with non-inverted (Fig.7a) setup (650 μM and 620 μM).

***Table 1***. $IC_{50}$ values of the molecules inhibiting the mannose/Concanavalin A interaction determined via SCP-RICM measurements and compared to SPR measurements

| Inhibitor | $IC_{50}$ value determined using SPR inhibition assay [μM] | $IC_{50}$ value determined using an inventive method [μM] |
|---|---|---|
| D-glucose | n.a. | $1.3 \cdot 10^5 \pm 1.9 \cdot 10^4$ |
| D-mannose | 6500 | 6491.4 ± 503.8 |
| $\alpha$-methyl-D-mannose | 750 | 617.9 ± 19.1 |
| $\alpha$-methyl-D-mannose[a] | 750 | 648.7 ± 76.8 |
| PAA-Man1 | 8 | 6.1 ± 0.7 |
| PAA-Man2 | 10 | 3.4 ± 0.5 |
| PAA-Man3 | 3 | 0.8 ± 0.1 |

[a] Measured with the inverted system PEG-ConA particles against a mannose functionalized surface. The $IC_{50}$ values for PAA Man are normalized per mannose, i.e. each mannose of PAA-Man3 has a 2.5-times larger affinity as compared to the mannose of PAA.Man1.

**Example 5: Regeneration of coverslips with immobilized ConA**

Mannose functionalized hydrogel particles of example 2A were added to ConA functionalized coverslips and the contact radius and particle radius was measured. The interaction was inhibited with α-methyl-D-mannose (200 μL) (10 mg $mL^{-1}$) so that the particles were detached from the surface. Afterwards, the surfaces were carefully washed with lectin binding buffer so that the particles were not washed out and the contact radii and particle radii were measured. The interaction was inhibited again with $\alpha$-methyl-D-mannose and the cycle was repeated 5 times. A shift from the initial measurement to the first washing cycle is observed as the surface could not be fully recovered. Since all measurements were normalized with respect to the blank signal the slight shift on the adhesion energy did not affect the reproducibility of the analysis.

## Claims

1. Method for the detection and/or characterization of an analyte based on determining the alteration of mechanical deformation of hydrogel particles.

2. Method according to claim 1, wherein said method comprises the following steps:

   a) providing a deformable, target molecule functionalized hydrogel particle
   b) providing a solid carrier with immobilized ligand
   c) providing an analyte to be tested
   d) forming a complex between the deformable, target molecule functionalized hydrogel particle and the solid carrier with immobilized ligand
   e) addition of the analyte and
   f) determining the alteration of mechanical deformation of hydrogel particles of step a).

3. Method according to claim 1, wherein said method comprises the following steps:

a) providing a deformable, ligand functionalized hydrogel particle
b) providing a solid carrier with immobilized target molecule
c) providing an analyte to be tested
d) forming a complex between the deformable, target molecule functionalized hydrogel particle and the solid carrier with immobilized ligand
e) addition of the analyte and
f) determining the alteration of mechanical deformation of hydrogel particles of step a).

**4.** Method to any one of claims 1 - 3, wherein the hydrogel particle has a Young's modulus between 0.1 kPa and 100 kPa.

**5.** Method according to any one of claims 1 - 4, wherein the hydrogel particle has a diameter between 10 to 50 μm.

**6.** Method according to any one of claims 1 - 5, wherein the hydrogel particles are made of polyethylene glycol which is functionalized with $-CH_2-COOH$, $-CH_2-CO-NH_2$, $-CH_2-CO-NH-CH=CH_2$, $-CH_2-CO-NH-C=CH$ or

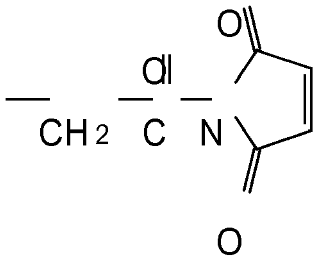

.

**7.** Method according to any one of claims 1 - 6, wherein the solid carrier is selected from the group comprising or consisting of: polymeric slide, a cover slip, a microtitre plate, cuvette, micro array slides, test tubes, and microfluidic chambers.

**8.** Method according to any one of claims 1 - 7, wherein step f) comprises optical determining the contact radius of the deformable, target molecule functionalized hydrogel particle bound to the solid carrier with immobilized ligand.

**9.** Use of the method according to any one of claims 1 - 8 in medical diagnosis.

**10.** Method according to any one of claims 1 - 9, wherein step f) comprises photometrically determining a change in brightness.

**11.** Method according to any one of claims 1 - 10, for high throughput screening.

**12.** Method according to any one of claims 1 - 11, wherein the ligand is chosen from the group comprising: ligand, substrate, antigen, signaling molecule, subunit of a protein complex, neurotransmitter, pheromone, toxin, toxoid, drug, second messenger, transcription factor, and metabolite.

**13.** Method according to any one of claims 1 - 12, wherein the target molecule is chosen from the group comprising: receptors, antibodies, enzymes, subunit of a protein complex, response element, DNA, ligand, chelator, peptide.

**14.** Method according to any one of claims 1 - 13, wherein the analyte is chosen from the group comprising: antibodies, pharmaceuticals, antibiotics, cytostatics, psychoactive substances, narcotics, analgesics, cardiac drugs, metabolites, coagulation inhibitors, hormones, interleukins, cytokines, performance-enhancing drugs, drugs, toxins, molecules derived from pathogens, viruses, prions, bacteria or parasites, noxious substances, pesticides, insecticides, wood preservatives, herbicides, fungicides, explosives, sugars, vitamins and flavors.

**15.** Kit for the detection and/or characterization of an analyte containing at least

a) a deformable, target molecule functionalized hydrogel particle
b) a solid carrier with immobilized ligand

Figure 1

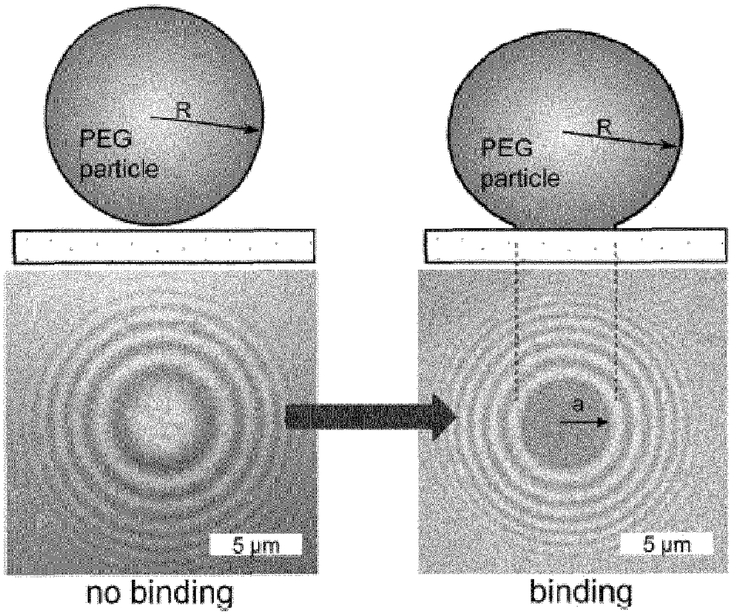


Figure 2

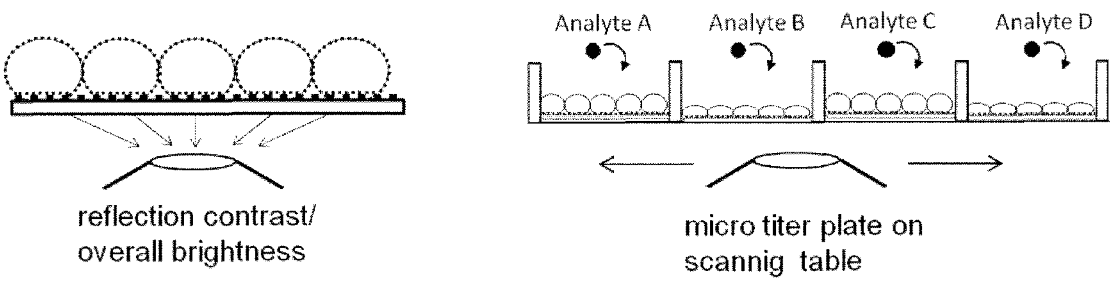

Figure 3

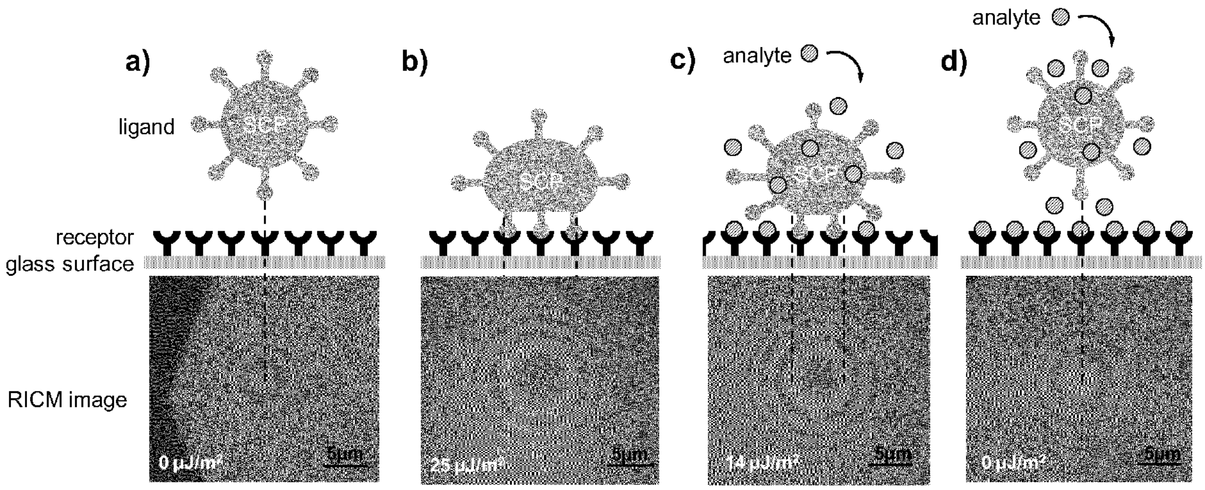
a)
b)
c)
d)
ligand
SCP
analyte
receptor
glass surface
RICM image
0 µJ/m²
25 µJ/m²
14 µJ/m²
0 µJ/m²
5µm

Figure 4

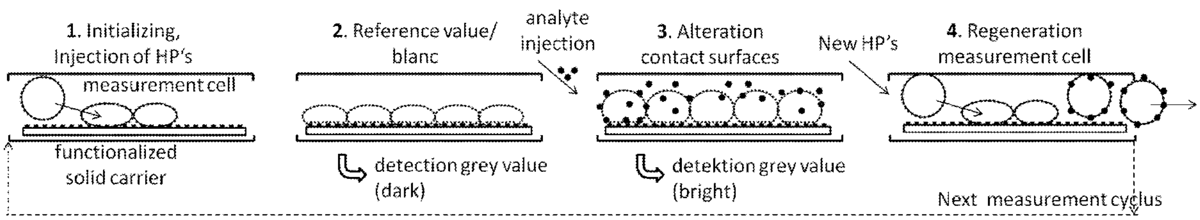


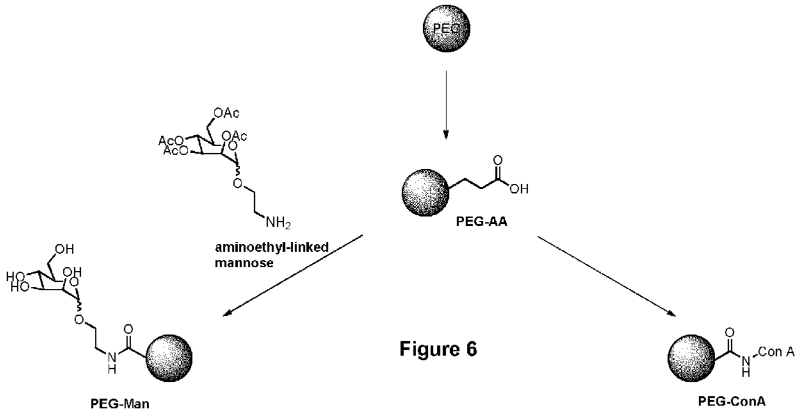

aminoethyl-linked mannose

PEG-AA

PEG-Man

PEG-ConA

Figure 6

**Figure 5**

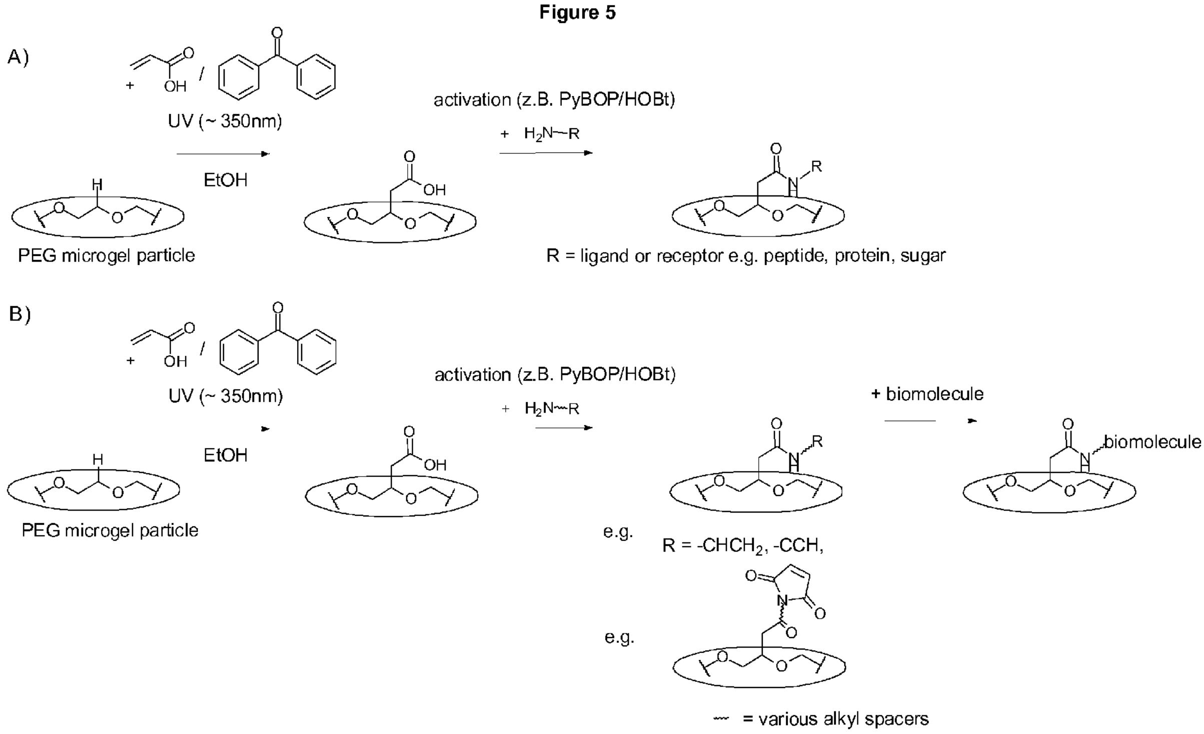
A)
UV (~ 350nm)
EtOH
activation (z.B. PyBOP/HOBt)
+ $H_2N$~R
PEG microgel particle
R = ligand or receptor e.g. peptide, protein, sugar
B)
UV (~ 350nm)
EtOH
activation (z.B. PyBOP/HOBt)
+ $H_2N$~R
+ biomolecule
biomolecule
PEG microgel particle
e.g.
R = -$CHCH_2$, -CCH,
e.g.
~ = various alkyl spacers

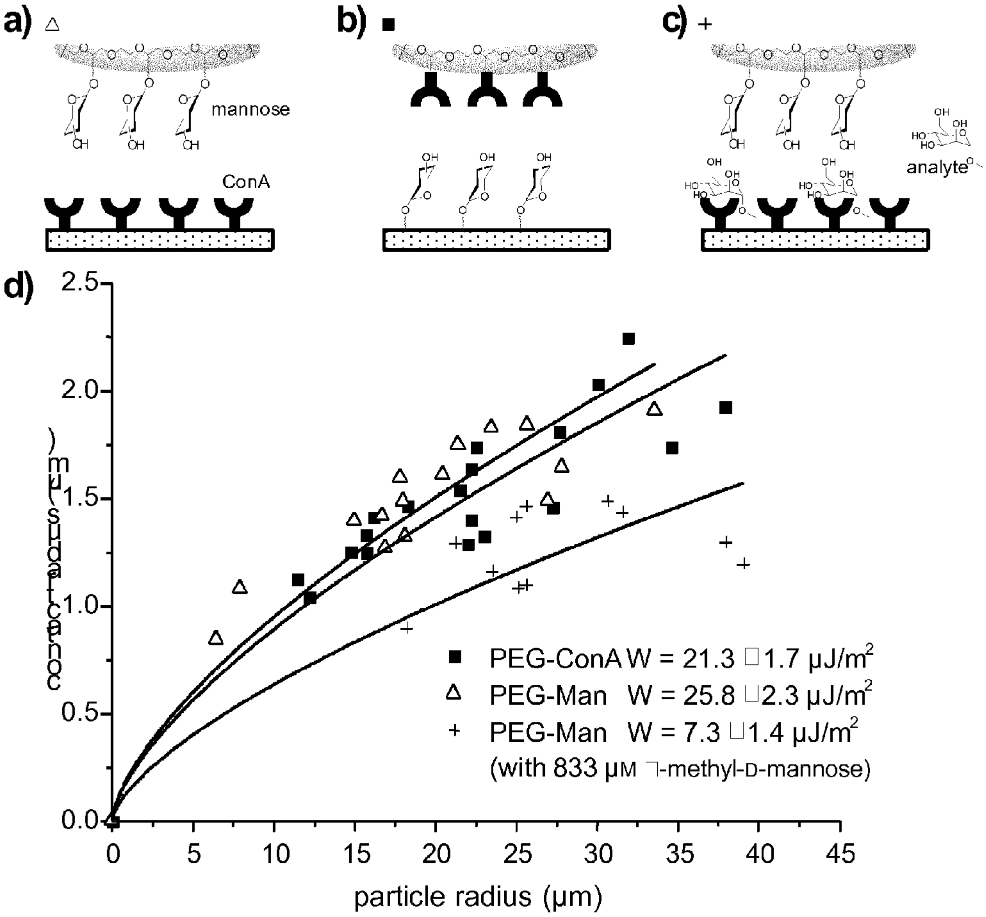
a)
mannose
ConA
b)
c)
analyte
d)
PEG-ConA W = 21.3 1.7 µJ/m²
PEG-Man W = 25.8 2.3 µJ/m²
PEG-Man W = 7.3 1.4 µJ/m²
(with 833 µM -methyl-D-mannose)
particle radius (µm)

Figure 7

Figure 8

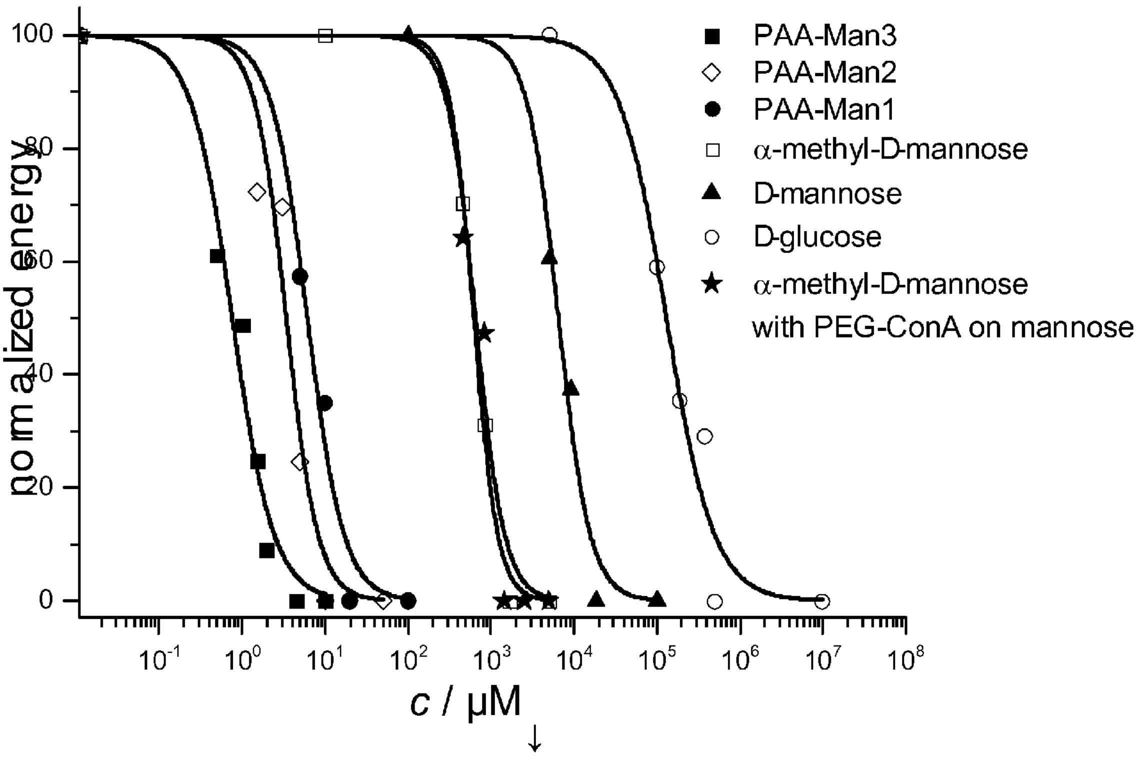
100
80
60
40
20
0
normalized energy
10-1 100 10[1] 10[2] 10[3] 10[4] 10[5] 10[6] 10[7] 10[8]
c / µM
PAA-Man3
PAA-Man2
PAA-Man1
α-methyl-D-mannose
D-mannose
D-glucose
α-methyl-D-mannose
with PEG-ConA on mannose

Europäisches Patentamt
European Patent Office
Office européen des brevets

# EUROPEAN SEARCH REPORT

Application Number
EP 13 15 0432

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GAWEL K ET AL: "Responsive hydrogels for label free signal transduction within biosensors", SENSORS, vol. 10, 30 April 2010 (2010-04-30), pages 4381-4409, XP002697114, ISSN: 1424-8220, DOI: 10.3390/s100504381 * the whole document * ----- | 1-15 | INV. G01N33/543 |
| A | GALAEV IGOR YU ET AL: "Effect of matrix elasticity on affinity binding and release of bioparticles. Elution of bound cells by temperature-induced shrinkage of the smart macroporous hydrogel.", LANGMUIR : THE ACS JOURNAL OF SURFACES AND COLLOIDS 2 JAN 2007, vol. 23, no. 1, 2 January 2007 (2007-01-02), pages 35-40, XP002697115, ISSN: 0743-7463 * abstract; figure 5 * ----- | 1-15 | |
| | | | TECHNICAL FIELDS SEARCHED (IPC) |
| A | DAINAIK M B ET AL: "Detachment of affinity-captured bioparticles by elastic deformation of a macroporous hydrogel", PROCEEDINGS OF THE NATIONAL ACADEMY OF SCIENCES, NATIONAL ACADEMY OF SCIENCES, US, vol. 103, no. 4, 24 January 2006 (2006-01-24), pages 849-854, XP003000336, ISSN: 0027-8424, DOI: 10.1073/PNAS.0508432103 * abstract; figure 2 * ----- | 1-15 | G01N |

1 The present search report has been drawn up for all claims

| Place of search | Date of completion of the search | Examiner |
|---|---|---|
| The Hague | 16 May 2013 | Routledge, Brian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 90011510 A


### Non-patent literature cited in the description


- **Y. YANASE et al.** *Biosens. Bioelectron.,* 2007, vol. 22, 1081-1086
- **M. SAITAKIS et al.** *Cell. Mol. Life Sci.,* 2012, vol. 69, 357-437
- **P. PATTNAIK.** *Applied Biochemistry and Biotechnology,* 2005, vol. 126, 79-92
- **Z. SHEN et al.** *Analytical Chemistry,* 2007, vol. 79, 2312-2319
- **J. S. DANIELS et al.** *Electroanalysis,* 2007, vol. 19, 1239-1257
- **HERTZ, H.** Ueber die Berührung fester elastischer Körper. Journal für die reine und angewandte Mathematik. *Crelles Journal,* vol. 1882 (92), 156-171